# EUROPEAN PATENT APPLICATION

(11) **EP 3 604 505 A1**
(43) Date of publication of application: **05.02.2020**
(21) Application number: 18776149.9
(22) Date of filing: 29.03.2018
(51) Int. Cl.: C12N 9/02, C07K 14/195, C12N 1/21, C12N 15/53, C12P 13/04

(54) **NOVEL MONOOXYGENASE AND UTILIZATION THEREOF**

(30) Priority: 31.03.2017 JP 2017071338
(71) Applicant: Kaneka Corporation, Osaka-shi, Osaka 530-8288 (JP); Kyoto University, Kyoto-shi, Kyoto 606-8501 (JP)
(72) Inventor: NOJIRI, Masutoshi, Takasago-shi Hyogo 676-8688 (JP); YASOHARA, Yoshihiko, Takasago-shi Hyogo 676-8688 (JP); HIBI, Makoto, Kyoto-shi Kyoto 606-8501 (JP); OGAWA, Jun, Kyoto-shi Kyoto 606-8501 (JP)
(74) Representative: Hoffmann Eitle
(86) International application number: PCT/JP2018/013363
(87) International publication number: WO 2018/181774

(57) **Abstract**

The present invention has an object of providing a novel enzyme protein that allows production of an optically active β-hydroxyamino acid. The present invention attains the above object by providing a monooxygenase including two kinds of hetero (different) subunits.

### [Sequence Listing]

SEQUENCE LISTING

<110>  KANEKA CORPORATION
       KYOTO UNIVERSITY

<120>  A novel monooxygenase and use thereof

<130>  B161123

<150>  JP 2017-071338
<151>  2017-03-31

<160>  23

<170>  PatentIn version 3.5

<210>  1
<211>  378
<212>  PRT
<213>  Rhodococcus wratislaviensis

<400>  1

Met Thr Ile Ile Glu His Gly Ser Leu Gly Thr Leu Pro Ala Pro Ser
1               5                   10                  15

Val Thr Thr Gly Ile Val Asp Ala Asp Ile His Pro Val Pro Gln Asp
            20                  25                  30

Gly Ala Leu Glu Pro Tyr Leu Asp Asp Arg Trp Lys Lys His Ile Arg
        35                  40                  45

Glu Tyr Gly Val Arg Thr Thr Thr Gly Leu Gln Phe Ile Ser Glu Tyr
    50                  55                  60

Pro Gln Met Tyr Gly Gly Ala Met Arg Ala Asp Ala Trp Pro Glu Ser
65                  70                  75                  80

Gly Tyr Pro Gly Ser Asp Arg Glu Leu Leu Arg Thr Gln Leu Leu Asp
                85                  90                  95

Lys His Asn Ile Gln Leu Gly Val Leu Gln Cys Leu Ala Pro Gly Gly
            100                 105                 110

Gln Thr Leu Asn Pro Ala Gly Gln Ala Leu Asn Gln Glu Leu Ala Ala
        115                 120                 125

Ala Leu Cys Arg Ala Thr Asn Asp Trp Gln Leu Glu His Leu Val Tyr
    130                 135                 140

Pro Asp Pro Arg Met Arg Ala Ala Ile Pro Val Thr Phe Glu Thr Pro
145                 150                 155                 160

Asp Tyr Ala Val Ala Glu Ile Glu Arg Val Gly Ala Asp Pro Gly Val
                165                 170                 175

Val Ala Val Leu Gly Thr Ser Lys Thr Leu Glu Pro Leu Gly Ser Arg
            180                 185                 190

Lys Tyr Trp Pro Ile Tyr Glu Ala Ser Val Ala Gln Asn Leu Pro Ile
        195                 200                 205

Gln Phe His Leu Ser Gln Gly Gly Gly His Ala Asn Thr Gly Thr Gly
    210                 215                 220

Trp Thr Ser Tyr His Thr Glu Tyr His Thr Gly His Val Gln Ser Phe
225                 230                 235                 240

Gln Ser Gln Leu Leu Ser Leu Val Leu Ser Gly Thr Phe Asp Arg Phe
                245                 250                 255

Pro Thr Leu Lys Val Met Phe Val Glu Gly Asn Val Ala His Phe Ala
            260                 265                 270

Pro Leu Ile Gln Arg Met Asp Tyr Thr Trp Glu Thr Leu Arg Gly Glu
        275                 280                 285

Leu Pro Asp Leu Gln Arg Lys Pro Ser Glu Tyr Ile Arg Asp His Ile
    290                 295                 300

Trp Ala Ser Thr Gln Pro Ile Asp Glu Pro Glu Lys Pro Glu His Leu
305                 310                 315                 320

Ala Glu Leu Leu Glu Glu Phe Cys Gly Asp Asn Val Val Phe Ala Thr
                325                 330                 335

Asp Tyr Pro His Phe Asp Phe Asp Asp Pro Glu Thr Ala Phe Pro Arg
            340                 345                 350

Ser Phe Pro Val Asp Leu Arg Asp Lys Ile Leu Arg Gly Asn Gly Met
        355                 360                 365

Arg Phe Phe Gly Val Thr Asn Gln Ala Asp
    370                 375

<210>  2
<211>  373
<212>  PRT
<213>  Rhodococcus wratislaviensis

<400>  2

Met Val Ala Pro Thr Ser Asn Pro Gly Val Pro Asp Glu Leu Asp Gly
1               5                   10                  15

Val Pro Ala Val Val Asp Cys Asp Val His Ala Val Leu Pro Ser Pro
            20                  25                  30

His Ser Leu Ile Pro Tyr Leu Asp Glu Tyr Trp Ala Asp Gln Leu Val
        35                  40                  45

Ala Gln Leu Ala Pro Thr Tyr Glu Pro Asn Tyr His Pro Arg Gly Ser
    50                  55                  60

Ala Ile Ala Gln His Ser Asp Ala Ser Val Asp Glu Asn Gly Arg Ala
65                  70                  75                  80

Ala Thr Thr Ala Glu Asn Leu Val Lys Asp Val Phe Ala Asp Gly Phe
                85                  90                  95

Thr Asp Phe Ala Val Val Asn Cys Leu Tyr Gly Val Gln Gln Ile His
            100                 105                 110

Gln Pro Arg Arg Glu Met Ala His Ala Arg Ala Leu Asn His Trp Ile
        115                 120                 125

Ala Asn Glu Trp Leu Asp Lys Asp Asp Arg Leu Arg Ala Ser Ile Val
    130                 135                 140

Val Pro Gln Gly Ser Pro Arg Ala Ala Ala Glu Glu Ile Asp Phe Trp
145                 150                 155                 160

Ser Gly Asp Lys Arg Phe Val Gln Val Leu Leu Leu Gly Gln Ser Glu
                165                 170                 175

Leu Leu Tyr Gly Arg Glu Ile Asn Trp Pro Ile Trp Glu Ala Ala Glu
            180                 185                 190

Ala Ala Gly Leu Pro Val Thr Leu His Ile Gly Gly Val Phe Arg Gln
        195                 200                 205

Ala Pro Thr Ser Val Gly Trp Pro Ala Ser His Leu Glu Trp Tyr Val
    210                 215                 220

Gly Gln Gln Ser Asn Ile Glu Ala Gln Leu Asn Ser Ile Ile Ser Glu
225                 230                 235                 240

Gly Ile Leu Gln Lys Phe Pro Lys Thr Lys Ile Leu Leu Ser Glu Leu
                245                 250                 255

Gly Phe Asn Trp Leu Pro Pro Phe Met Trp Lys Phe Asp Lys Leu Trp
            260                 265                 270

Lys Ser Tyr Arg Pro Asp Ile Pro Trp Val Gln Glu Ser Pro Leu Glu
        275                 280                 285

Leu Ile Arg Glu His Val Arg Val Thr Thr Ser Pro Ser Asp Gly Ala
    290                 295                 300

Glu Glu Ala Gly Arg Leu Asp Ser Ile Val Asp Arg Leu Gly Ser Asp
305                 310                 315                 320

Arg Met Leu Val Tyr Ser Ser Asp Tyr Pro His Lys His His Ser Gly
                325                 330                 335

Pro Arg Asp Ile Glu Asn Gly Thr His Ser Pro Glu Leu Leu Asp Arg
            340                 345                 350

Ile Tyr Arg Arg Asn Ala Phe Asp Leu Tyr Asn Leu Val Val Pro Ser
        355                 360                 365

Pro Gly Lys Val Gly
    370

<210> 3
<211> 322
<212> PRT
<213> Rhodococcus wratislaviensis

<400> 3

Met Thr Asn Ser Asp Ser Ser Asp Ala Val Ala Glu Val Asp Val Ile
1               5                   10                  15

Ile Asp Gly Ile Thr Asn Glu Ala Asp Gly Ile Ile Ser Leu Thr Phe
            20                  25                  30

Ala Ser Leu Thr Gly Asp Lys Met Pro Arg Trp Glu Pro Gly Ala His
        35                  40                  45

Ile Asp Val Leu Leu Gly Pro Glu Leu Glu Arg Gln Tyr Ser Leu Cys
    50                  55                  60

Gly Asp Val Asn Asp Glu Arg Thr Leu Arg Val Ala Val Leu Arg Glu
65                  70                  75                  80

Pro Asp Ser Arg Gly Gly Ser Gln Trp Val His Glu Arg Leu Ser Val
                85                  90                  95

Gly Asp Lys Ile Arg Ile Arg Gly Pro Arg Asn His Phe Arg Leu Val
            100                 105                 110

Glu Ala Gln Glu Tyr Leu Phe Ile Ala Gly Gly Ile Gly Ile Thr Pro
        115                 120                 125

Ile Leu Pro Met Ile Ala Asp Cys Glu Ala Arg Gly Ser Arg Trp Ser
    130                 135                 140

Leu Val Tyr Gly Gly Arg Ser Thr Asp Ser Met Ala Phe Thr Thr Gln
145                 150                 155                 160

Leu Ala Lys His Gln Asp Arg Val Arg Phe Trp Pro Gln Asp Glu Asn
                165                 170                 175

Gly His Ile Asp Ile Arg Gly Phe Leu Gly Asp Pro Arg Pro Gly Thr
            180                 185                 190

Ala Ile Tyr Cys Cys Gly Pro Gly Pro Leu Leu Asp Ala Val Glu Asp
        195                 200                 205

Val Cys Ser Gln Trp Pro Thr Asp Ala Leu His Leu Glu Arg Phe Arg
    210                 215                 220

Pro Arg Ala Gly Ala Met Glu Gly Pro Glu Ser Pro Phe Glu Val Glu
225                 230                 235                 240

Leu Glu Gln Ser Asn Met Val Val Thr Val Ala Val Gly Gln Thr Ile
                245                 250                 255

Val Asp Ala Cys Lys Ser Ala Gly Val His Ile Pro Thr Ser Cys Gly
            260                 265                 270

Glu Gly Thr Cys Gly Thr Cys Glu Thr Val Val Leu Glu Gly Val Pro
        275                 280                 285

Glu His Arg Asp Ser Tyr Leu Thr Ala Gln Glu Arg Glu Ser Asn Glu
    290                 295                 300

Val Val Met Pro Cys Cys Ser Arg Ser Arg Thr Ser Arg Leu Val Leu
305                 310                 315                 320

Asp Leu

<210>  4
<211>  169

<212>  PRT
<213>  Rhodococcus wratislaviensis

<400>  4

Met Ser Lys Phe Val Ile Gly Ser Val Asn Asp Ile Pro Pro Gly Ser
1               5                   10                  15

Arg Lys Ile Val Thr Leu Asp Gly Arg Ser Val Gly Ile Phe Asn Leu
            20                  25                  30

Asp Gly Asp Tyr Phe Ala Leu Leu Asn Gln Cys Pro His Lys Gly Ala
        35                  40                  45

Glu Val Cys Ser Thr Gly Thr Ile Phe Gly Ile Ser His Ala Asp Lys
    50                  55                  60

Pro Asn Glu Pro Ile Ala Tyr Glu Arg Glu Arg Ser Ile Arg Cys Pro
65                  70                  75                  80

Trp His Gln Trp Glu Phe Asp Ile Arg Thr Gly Gln Ser Phe Tyr Asp
                85                  90                  95

Pro Lys Gly Glu Arg Ile Arg Lys Tyr Asn Val Ser Val Val Ala Gly
            100                 105                 110

Ala Glu Val Ala Gly Asp Ser Glu Glu Gly Val Lys Asn Gly Pro Tyr
        115                 120                 125

Val Met Glu Gly Tyr Glu Val Ser Val Glu Asp Asp Leu Val Val Val
    130                 135                 140

Asp Thr Ser Arg Arg Arg Ala Gly Val Gly Asn Asn Met Pro Thr Ala
145                 150                 155                 160

Asp Ala Ala Gln Thr Asp Leu Thr Val
                165


<210>  5
<211>  480
<212>  PRT
<213>  Rhodococcus wratislaviensis

<400>  5

Met Thr Val Asn His Ser His Glu Gln Leu Asn Glu Asp Gly Leu Ala
1               5                   10                  15

Gln Gly Leu Asn Lys Arg His Ile Arg Met Ile Ala Ile Gly Gly Ala
            20                  25                  30

Ile Gly Val Gly Leu Phe Leu Gly Ser Gly Lys Gly Ile Ser Tyr Ala
        35                  40                  45

Gly Pro Ala Val Ile Gly Val Tyr Ala Val Thr Gly Val Phe Ile Phe
    50                  55                  60

Ile Ile Met Arg Ala Leu Gly Glu Leu Leu Met Tyr Arg Pro Val Thr
65                  70                  75                  80

Gly Ser Phe Ala Glu Tyr Ala Arg Glu Phe Leu Gly Pro Phe Val Gly
                85                  90                  95

Phe Val Thr Gly Trp Gly Tyr Trp Phe Thr Trp Thr Ile Ile Gly Met
            100                 105                 110

Ala Glu Ile Thr Ala Ala Gly Ile Phe Ala Lys Phe Trp Phe Pro Glu
        115                 120                 125

Met Pro Gln Tyr Leu Thr Ala Leu Ile Ala Leu Ile Ala Ile Val Ile
    130                 135                 140

Leu Asn Leu Ala Lys Val Gly Phe Phe Gly Glu Ala Glu Phe Trp Phe
145                 150                 155                 160

Ala Ser Ile Lys Val Ile Ala Ile Val Ser Leu Ile Gly Ser Gly Ile
                165                 170                 175

Phe Met Leu Val Phe Arg Val Gly Ser Ala Gly Lys Glu Gly Ser Ile
            180                 185                 190

Thr Asn Leu Trp Asp His Gly Gly Met Ala Pro His Gly Leu Leu Ala
        195                 200                 205

Val Leu Leu Ala Phe Gln Ile Val Val Phe Ser Tyr Gln Gly Val Glu
    210                 215                 220

Leu Ile Gly Met Thr Ala Ala Glu Ala Lys Asp Arg Glu Asn Val Leu
225                 230                 235                 240

Pro Lys Ala Ile Asn Ser Ile Pro Trp Arg Ile Gly Val Phe Tyr Val
                245                 250                 255

Gly Thr Leu Ile Val Leu Leu Ser Leu Phe Pro Trp Thr Gln Phe Gly
            260                 265                 270

Pro Thr Glu Ser Pro Phe Val Lys Gly Phe Thr Gln Ile Gly Leu Pro
        275                 280                 285

Ala Ala Ala Ser Ile Met Asn Phe Val Val Leu Ala Ser Ala Leu Ser
    290                 295                 300

Ser Cys Ser Ala Gly Leu Tyr Ser Asn Gly Arg Leu Leu Lys Lys Leu
305                 310                 315                 320

Ala Ser Asp Gly Leu Ala Pro Lys Arg Phe Glu Lys Thr Asn Arg Gly
                325                 330                 335

His Val Pro Ala Ala Ala Ile Ile Ala Ser Gly Ala Leu Met Leu Val
            340                 345                 350

Gly Val Ala Ile Asn Ala Val Val Pro Glu Lys Ala Phe Ser Tyr Ile
        355                 360                 365

Ser Ser Val Ala Thr Leu Gly Ala Ile Trp Ser Trp Gly Val Ile Leu
    370                 375                 380

Val Cys His Leu Arg Tyr Arg Arg Arg Val Glu Gln Gly Glu Val Thr
385                 390                 395                 400

Glu Ser Ser Phe Lys Leu Pro Tyr Ala Lys Pro Leu Cys Trp Ser Thr
                405                 410                 415

Leu Ala Phe Leu Gly Gly Val Leu Val Leu Leu Ala Phe Asp Glu Ser
            420                 425                 430

Gln Arg Ile Ala Leu Tyr Ala Leu Pro Val Trp Ala Val Leu Phe Ala
        435                 440                 445

Gly Gly Tyr Tyr Phe Ser Ser Arg Ser Asn Arg Ser Gly Asn Gln Ala
    450                 455                 460

Asp Asp Arg Ala Pro Arg Asp Asp Gln Pro Val Leu Gln Pro Arg Ile
465                 470                 475                 480

<210>  6
<211>  1137
<212>  DNA
<213>  Rhodococcus wratislaviensis

<400>  6
atgaccatca tcgaacacgg tagcctcggt acgctgccgg cgccgagcgt gacgactggc      60

attgttgatg ccgacatcca cccggttccc caagacggtg cactggaacc gtacctggat     120

gatcgttgga agaagcacat ccgtgagtat ggtgtgcgga ccaccaccgg tttgcagttc     180

atcagtgagt acccgcagat gtacggcggc gccatgcgag ctgatgcatg gcctgagtcc     240

ggttaccctg gctcggaccg agagcttctc cggacccagc tgctggacaa gcacaacatc 300

cagctgggag ttctccagtg cctcgccccg ggaggccaga ccctgaaccc cgccggccag 360

gccctgaacc aggaacttgc ggcggcactg tgcagggcaa caaatgattg gcagctcgag 420

cacctcgtgt acccggaccc ccggatgcga gcggcaattc cggtcacgtt cgagacgccc 480

gactacgctg ttgctgagat cgagcgggtt ggagcggacc cgggcgtggt cgccgtactg 540

ggtacgagca agacgctcga accgctgggc agcaggaagt actggccgat ctacgaagcc 600

tcagtcgcgc agaacctgcc gattcagttc cacctgtccc agggaggtgg acacgccaac 660

acgggaactg ggtggacgtc ctaccacacg gaataccaca caggtcacgt tcagagcttc 720

cagtcgcagc tactgagcct ggtcctgtcg ggaacgttcg accgattccc cacgctcaag 780

gtgatgttcg tcgaaggaaa cgtcgctcat ttcgctccgc tcattcagcg aatggactac 840

acgtgggaga cgctccgagg tgagctgccc gaccttcagc ggaagccttc cgagtacatt 900

cgcgatcaca tctgggcatc gacgcagccg atcgatgagc cggagaagcc ggaacacctc 960

gccgagctcc tcgaggaatt ctgcggcgac aacgtcgtct tcgcaaccga ctacccgcat 1020

ttcgacttcg acgacccgga aaccgcattc ccgcgtagct tccccgtgga cctgcgggac 1080

aagattctcc gcggaaacgg tatgaggttc ttcggcgtta cgaatcaggc ggactaa 1137

<210> 7
<211> 1122
<212> DNA
<213> Rhodococcus wratislaviensis

<400> 7
atggttgcac caacctcgaa ccccggagtg cctgacgaac tggacggcgt ccccgccgtt 60

gtggactgcg acgtgcatgc cgtcctcccg tcgccccact cactgattcc gtatctcgat 120

gagtactggg ctgatcagct cgttgcacag ctcgcaccga cgtacgagcc gaactaccac 180

ccccgtggct cagcgattgc gcagcactcg gacgcgtcgg tcgatgagaa cggacgcgcg 240

gcgacgacgg ccgaaaatct ggtcaaagat gtattcgccg acggatttac tgatttcgcc 300

gttgttaact gcctctatgg cgtgcagcag attcatcagc ctcgacgcga gatggctcat 360

gcgcgcgctc tcaaccactg gatcgcgaac gagtggctcg acaaagacga ccggcttcgt 420

gcctcgattg tcgttcccca gggatcgccc cgtgctgcgg ccgaggaaat cgacttctgg 480

tccggcgata aacgattcgt ccaggttctc ctgctcgggc agtccgagct gctgtatggg 540

cgcgaaatca actggccgat ctgggaagcg gcggaggccg ccgggttgcc tgtcaccctt 600

cacatcggag gtgtctttcg gcaagccccg acgtcggttg gttggcctgc atcgcacctg 660

gagtggtatg tcggtcagca gtcgaacatc gaggcgcagc tgaactccat catctctgag 720

ggaatcctcc agaagtttcc gaagactaag attctgctca gcgaactcgg attcaactgg 780

cttcccccgt ttatgtggaa gttcgacaag ctatggaaga gctaccgccc ggacatcccc 840

tgggtacagg aatcaccgtt ggagctcatt cgggaacacg tccgggtgac gacgagccca 900

tcggatggcg ccgaggaagc gggacgactc gattcgatcg tggacaggtt ggggtcggac 960

cggatgctcg tgtactcgag cgactatccc cacaaacacc actccggacc ccgcgacatc 1020

gagaacggaa cgcacagtcc ggaacttcta gaccgcatct atcgacgtaa tgcattcgac 1080

ctctacaacc tcgttgtccc gagcccagga aaggttggtt aa 1122

<210> 8
<211> 969
<212> DNA
<213> Rhodococcus wratislaviensis

<400> 8
atgaccaatt cagatagttc ggacgccgtc gccgaggttg atgtgatcat cgatggaatc 60

accaacgagg cagatggaat tatttctctc acctttgcct cgttgaccgg agacaagatg 120

ccgcggtggg aacccggagc tcacatcgac gtgctattgg gccccgaact ggaacgacag 180

tactctctgt gtggtgatgt gaacgatgag cgcacgctac gcgtcgctgt tctgcgcgaa 240

ccggatagcc gaggcggctc tcagtgggtt cacgagcgcc tgagcgtcgg agacaagatc 300

cgtatcagag gaccacgcaa ccattttcgt ctggttgagg cccaagagta tctcttcatc 360

gccggcggaa tcggtatcac gccaatactg ccgatgatcg ctgactgcga ggcaagaggc 420

agccggtggt cgctcgtcta cggcggccga agtaccgatt ccatggcgtt caccacccaa 480

ctcgcaaagc atcaggaccg tgtcagattt tggccccagg acgagaacgg ccatatcgat 540

atccggggat ttctgggaga tcccagaccg ggaaccgcga tctactgttg cgggccagga 600

ccactcctcg atgcggtcga ggatgtgtgt agccaatggc caacggatgc actacatctc 660

gaacgattcc gtcccagagc gggcgcgatg gaaggcccgg agagtccgtt cgaggtcgaa 720

ctcgagcagt cgaatatggt ggtgactgtt gccgtcggtc agacgatcgt ggatgcatgc 780

aaatctgcgg gggtgcacat tccaacatct tgcggcgaag gtacctgtgg gacgtgtgaa 840

acggtcgtgt tagaaggtgt tcccgaacac cgggattcct accttacggc gcaagaaagg 900

gaatccaatg aggtagtcat gccgtgctgc tcgcgatcgc gaacgagtcg gctcgtcctc 960

gatctctag 969

<210> 9
<211> 510
<212> DNA
<213> Rhodococcus wratislaviensis

<400> 9
atgtccaagt ttgtaatcgg tagcgtaaac gacattccac caggatcccg gaagatcgta 60

accctcgatg gacggtccgt gggaatcttc aacctcgatg gtgactactt cgcgctcctc 120

aatcagtgtc cgcacaaggg cgcggaagtg tgctcgaccg gaaccatctt cggtatctcg 180

cacgcggaca agccaaatga gccgatcgcc tacgagcgag agcggtccat ccgttgtccc 240

tggcaccagt gggaattcga catccgtacg ggccagtcat tctatgaccc gaagggcgag 300

cggatccgca agtacaacgt gtcggttgtc gcgggcgccg aggtggccgg tgattccgaa 360

gaaggcgtga agaacggtcc gtatgtcatg gagggctacg aggtgtcggt cgaggatgac 420

ctggttgtcg tcgacacgag ccgacggcgc gcaggcgtgg gcaacaacat gcccaccgct 480

gatgccgcgc aaacggacct gacggtctga 510

<210> 10
<211> 1443
<212> DNA
<213> Rhodococcus wratislaviensis

<400> 10
atgacagtga atcattccca cgagcagttg aatgaggatg gcctagcgca gggccttaac 60

aagcgacata tccggatgat cgcgatcggc ggtgctatcg gtgtaggact tttcctcgga 120

tccggaaagg gaatctccta tgcgggcccc gcggtgatcg gtgtctacgc ggttaccgga 180

gtgttcattt tcatcatcat gagggctctc ggcgagctcc tgatgtaccg cccagtgacg 240

ggaagttttg ccgagtatgc gcgagaattt ctggggccat tcgtcgggtt cgtaaccggc 300

tggggatatt ggtttacctg gacaatcatt ggcatggcgg agatcaccgc agctgggatt 360

tttgccaaat tctggtttcc tgaaatgccg cagtacctca ctgcgctgat cgcgctgatt 420

gcgatcgtta tccttaatct ggctaaagtc ggcttcttcg gagaagccga gttctggttt 480

gcctcgatta aagtgattgc gatcgtgtct ctgatcggca gcggaatatt catgctcgta 540

ttccgggttg ggtcggccgg caaggaaggc agcattacga atctctggga tcacggtggc 600

atggcaccgc acggcctcct cgctgtgttg ttggctttcc agattgtggt cttctcgtac 660

cagggcgtcg agctgattgg catgactgca gcagaagcca aggaccggga gaacgttctc 720

ccgaaggcga tcaactctat cccgtggcga attggtgtct tctacgtcgg gactctgatc 780

gtgttgctct cgctttttcc ctggacccaa tttggaccca ctgaaagccc gttcgtgaag 840

ggattcacac agatcgggtt gccggctgcc gcgagtatca tgaacttcgt cgtcctggcc 900

tcggcgcttt cgtcctgcag cgccggcctc tacagcaacg gacggcttct caagaagttg 960

gcctccgatg gtctcgcgcc gaagcgattt gagaagacca atcgcgggca cgttcccgcg 1020

gccgccatca tcgcttcggg cgcgctgatg ctggttggtg tcgcaatcaa tgcagttgtt 1080

ccggagaagg cgttctcata catttcctcc gtcgccaccc tgggcgcaat ctggagttgg 1140

ggagtaattc tggtgtgcca cctccgctat cgacggcgcg tcgaacaagg cgaagtcacc 1200

gaaagctcct tcaagcttcc atacgccaag ccgctttgct ggagcaccct cgccttcctc 1260

ggcggtgtcc ttgtgctcct tgccttcgac gaaagccagc gcatcgccct ttacgccttg 1320

ccggtatggg cagtactttt cgccggcggc tactactttt cgtcgcggag taaccggtcc 1380

ggcaatcagg ctgatgatcg cgctccacga gatgaccagc ctgttctgca gcccagaatc 1440

tga 1443

<210> 11
<211> 47
<212> DNA
<213> Artificial Sequence

<220>
<223> Primer sequence

<400> 11
tgtttaactt taagaaggag atataccatg gttgcaccaa cctcgaa 47

<210> 12
<211> 47
<212> DNA
<213> Artificial Sequence

<220>
<223> Primer sequence

<400> 12
tggtgatggt gatgctcgag agatctacta gagatcgagg acgagcc 47

<210> 13
<211> 47
<212> DNA
<213> Artificial Sequence

<220>
<223> Primer sequence

<400> 13
tgtttaactt taagaaggag atataccatg accatcatcg aacacgg 47

<210> 14
<211> 47
<212> DNA
<213> Artificial Sequence

<220>
<223> Primer sequence

<400> 14
cgctaccgat tacaaacttg gacattctta accaaccttt cctgggc 47

<210> 15
<211> 47
<212> DNA
<213> Artificial Sequence

<220>
<223>  Primer sequence

<400>  15
cccgagccca ggaaaggttg gttaagaatg tccaagtttg taatcgg                    47


<210>  16
<211>  47
<212>  DNA
<213>  Artificial Sequence

<220>
<223>  Primer sequence

<400>  16
gaattcatta aagaggagaa attaaccatg gttgcaccaa cctcgaa                    47


<210>  17
<211>  47
<212>  DNA
<213>  Artificial Sequence

<220>
<223>  Primer sequence

<400>  17
caacaggagt ccaagctcag ctaattacta gagatcgagg acgagcc                    47


<210>  18
<211>  47
<212>  DNA
<213>  Artificial Sequence

<220>
<223>  Primer sequence

<400>  18
gtttaacttt aagaaggaga tatacatatg acagtgaatc attccca                    47


<210>  19
<211>  47
<212>  DNA
<213>  Artificial Sequence

<220>
<223>  Primer sequence

<400>  19
tggtgatggt gatgctcgag agatctatca gattctgggc tgcagaa                    47


<210>  20
<211>  47
<212>  DNA
<213>  Artificial Sequence

<220>
<223>  Primer sequence

<400>  20
tcgcatcacc atcaccatca cggatccatg gttgcaccaa cctcgaa                    47


<210>  21
<211>  47
<212>  DNA
<213>  Artificial Sequence

<220>
<223>  Primer sequence

<400>  21
caacaggagt ccaagctcag ctaattatta gtccgcctga ttcgtaa                    47


<210>  22
<211>  47
<212>  DNA
<213>  Artificial Sequence

<220>
<223>  Primer sequence

<400>  22
tcgcatcacc atcaccatca cggatccatg accaattcag atagttc                    47


<210>  23
<211>  47
<212>  DNA
<213>  Artificial Sequence

<220>
<223>   Primer sequence

<400>  23
caacaggagt ccaagctcag ctaattacta gagatcgagg acgagcc                    47

## Description

### Technical Field

The present invention relates to a novel monooxygenase, a gene encoding the same, and use thereof.

### Background Art

An optically active α,α-disubstituted amino acid increases the stability against a peptidase, and is promising as a building block of a peptide pharmaceutical drug (see Non-Patent Literature 1).

An optically active α,α-disubstituted amino acid may be produced by, for example, a method disclosed in Non-Patent Literature 2, that is, a method for producing an optically active α,α-disubstituted amino acid through asymmetric alkylation involving use of chiral phase-transfer catalysts. Further, Patent Literature 1 discloses a reaction for synthesizing α-methyl-L-serine through an enzymatic reaction based on L-alanine and formaldehyde as ingredients. Citation List

### [Patent Literature]

[Patent Literature 1]
Japanese Patent No. 4877227 (Publication Date: February 15, 2012)

### [Non-patent Literature]

[Non-patent Literature 1]
   Claudio Mapelli et al., J. Med. Chem., 2009, 52, 7788-7799.
[Non-patent Literature 2]
   Takashi Ooi et al., J. Am. Chem. Soc., 2003, 125, 5139-5151

### Summary of Invention

### Technical Problem

The method disclosed in Non-Patent Literature 2 allows an optically active α,α-disubstituted amino acid to be produced, but unfortunately requires complicated steps such as introduction of a protecting group and deprotecting after reaction. The method disclosed in Patent Literature 1 does not require a protecting group, but only allows α-methyl-L-serine (L form) to be synthesized, and does not allow α-methyl-D-serine (which is an optical isomer of α-methyl-L-serine) to be synthesized.

There has thus been a demand for an improved method for simply and efficiently producing an optically active α,α-disubstituted amino acid, for example, optically active α-methyl-D-serine.

In view of the above, it is an object of the present invention to provide a novel enzyme protein that allows production of a β-hydroxyamino acid including an optically active α,α-disubstituted amino acid, for example, production of α-methyl-D-serine.

### Solution to Problem

The inventor of the present invention conducted diligent research for attaining the above object, and thereby succeeded in isolating a microorganism in the natural world which microorganism has the ability to convert 2-aminoisobutyric acid into optically active α-methyl-D-serine. The inventor also succeeded in, through proteome analysis and various analyses related thereto of the microorganism, identifying a novel monooxygenase for converting 2-aminoisobutyric acid into α-methyl-D-serine. Further, as the inventor analyzed the identified novel monooxygenase in detail, the inventor discovered that even in a case where the substrate is L-isovaline, D-isovaline, L-aminobutyrate, D-aminobutyrate, or the like, the monooxygenase has activity for conversion of an α-amino acid or α,α-disubstituted amino acid into an optically active β-hydroxyamino acid. The inventor has thus completed the present invention. Therefore, the present invention has the aspects below.
[1] A monooxygenase, including: two kinds of hetero subunits.
[2] The monooxygenase according to [1], wherein
   the monooxygenase catalyzes a reaction for converting an α-amino acid or α,α-disubstituted amino acid into a β-hydroxyamino acid.
[3] The monooxygenase according to [2], wherein
   the α-amino acid or α,α-disubstituted amino acid is a compound represented by Formula (1) below: 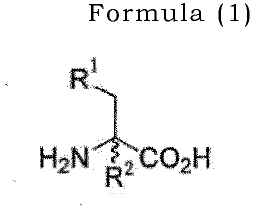
   where R¹ and R² are each independently a hydrogen or CH₃, and
   the β-hydroxyamino acid is a compound represented by Formula (2) below: 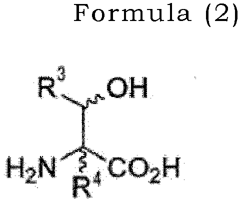
   where R³ and R⁴ are each independently a hydrogen or CH₃.
[4] The monooxygenase according to any one of [1] to [3], wherein
   the monooxygenase catalyzes at least one of respective reactions represented by Formulae (3) to (7) below which reactions are each for converting the α-amino acid or α,α-disubstituted amino acid into the β-hydroxyamino acid, 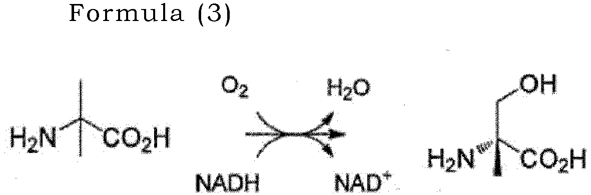 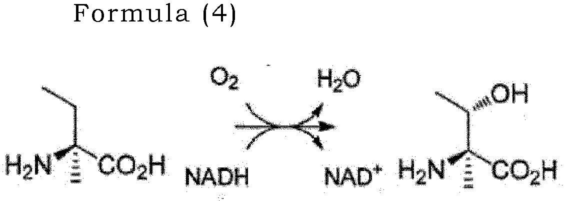 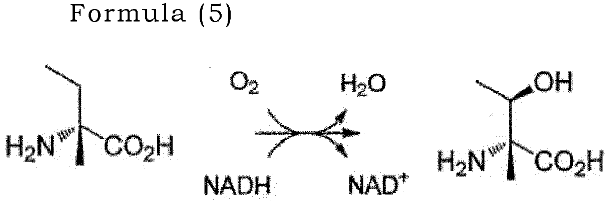 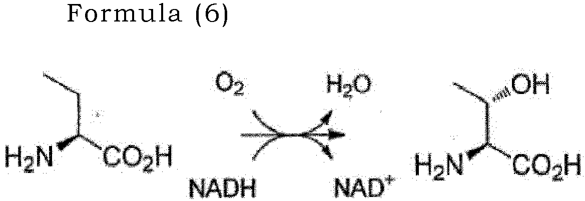 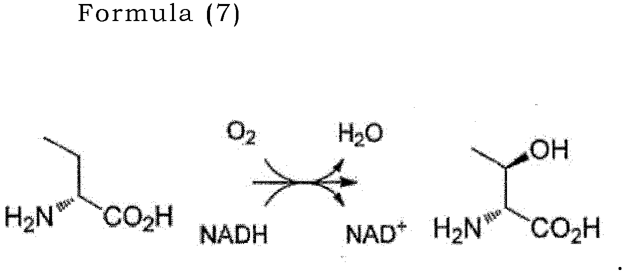
[5] The monooxygenase according to any one of [1] to [4], wherein
   assuming that the two kinds of hetero subunits are an a subunit and a β subunit,
   the a subunit includes a protein selected from the group consisting of (a) to (d) below, and
   the β subunit includes a protein selected from the group consisting of (e) to (h) below,
   (a) a protein consisting of an amino acid sequence represented by SEQ ID NO: 1;
   (b) a protein derived from the amino acid sequence of SEQ ID NO: 1 by substitution, deletion, insertion, and/or addition of one (1) or several amino acid residues, a subunit including the protein (b) having monooxygenase activity in a case where the subunit including the protein (b) has formed a complex together with the β subunit;
   (c) a protein consisting of an amino acid sequence having a homology of not less than 80% with respect to the amino acid sequence represented by SEQ ID NO: 1, a subunit including the protein (c) having monooxygenase activity in a case where the subunit including the protein (c) has formed a complex together with the β subunit;
   (d) a protein encoded by a gene consisting of a base sequence represented by SEQ ID NO: 6;
   (e) a protein consisting of an amino acid sequence represented by SEQ ID NO: 2;
   (f) a protein derived from the amino acid sequence of SEQ ID NO: 2 by substitution, deletion, insertion, and/or addition of one (1) or several amino acid residues, a subunit including the protein (f) having monooxygenase activity in a case where the subunit including the protein (f) has formed a complex together with the a subunit;
   (g) a protein consisting of an amino acid sequence having a homology of not less than 80% with respect to the amino acid sequence represented by SEQ ID NO: 2, a subunit including the protein (g) having monooxygenase activity in a case where the subunit including the protein (g) has formed a complex together with the a subunit, and
   (h) a protein encoded by a gene consisting of a base sequence represented by SEQ ID NO: 7.
[6] A monooxygenase reaction system for producing a β-hydroxyamino acid, the system including: a monooxygenase according to any one of [1] to [5]; and an electron transport chain protein.
[7] The monooxygenase reaction system according to [6], wherein
   the electron transport chain protein includes a protein selected from the group consisting of (i) to (1) below and a protein selected from the group consisting of (m) to (p) below,
   (i) a protein consisting of an amino acid sequence represented by SEQ ID NO: 3;
   (j) a protein derived from the amino acid sequence of SEQ ID NO: 3 by substitution, deletion, insertion, and/or addition of one (1) or several amino acid residues, the protein (j) having electron transport activity in a case where the protein (j) has been combined with a protein selected from the group consisting of (m) to (p) below;
   (k) a protein consisting of an amino acid sequence having a homology of not less than 80% with respect to the amino acid sequence represented by SEQ ID NO: 3, the protein (k) having electron transport activity in a case where the protein (k) has been combined with a protein selected from the group consisting of (m) to (p) below;
   (l) a protein encoded by a gene consisting of a base sequence represented by SEQ ID NO: 8;
   (m) a protein consisting of an amino acid sequence represented by SEQ ID NO: 4;
   (n) a protein derived from the amino acid sequence of SEQ ID NO: 4 by substitution, deletion, insertion, and/or addition of one (1) or several amino acid residues, the protein (n) having electron transport activity in a case where the protein (n) has been combined with a protein selected from the group consisting of (i) to (l) above;
   (o) a protein consisting of an amino acid sequence having a homology of not less than 80% with respect to the amino acid sequence represented by SEQ ID NO: 4, the protein (o) having electron transport activity in a case where the protein (o) has been combined with a protein selected from the group consisting of (i) to (l) above; and
   (p) a protein encoded by a gene consisting of a base sequence represented by SEQ ID NO: 9.
[8] A monooxygenase gene encoding a protein recited in any one of [1] to [5].
[9] A recombinant vector, including: a gene according to [8].
[10] A transformant, including: a gene according to [8]; or a recombinant vector according to [9].
[11] The transformant according to [10], further including:
   (1) a gene encoding an electron transport chain protein; and/or
   (2) a gene encoding a transporter protein.
[12] The transformant according to [11], wherein
   the electron transport chain protein includes a protein selected from the group consisting of (i) to (l) below and a protein selected from the group consisting of (m) to (p) below,
   (i) a protein consisting of an amino acid sequence represented by SEQ ID NO: 3;
   (j) a protein derived from the amino acid sequence of SEQ ID NO: 3 by substitution, deletion, insertion, and/or addition of one (1) or several amino acid residues, the protein (j) having electron transport activity in a case where the protein (j) has been combined with a protein selected from the group consisting of (m) to (p) below;
   (k) a protein consisting of an amino acid sequence having a homology of not less than 80% with respect to the amino acid sequence represented by SEQ ID NO: 3, the protein (k) having electron transport activity in a case where the protein (k) has been combined with a protein selected from the group consisting of (m) to (p) below;
   (l) a protein encoded by a gene consisting of a base sequence represented by SEQ ID NO: 8;
   (m) a protein consisting of an amino acid sequence represented by SEQ ID NO: 4;
   (n) a protein derived from the amino acid sequence of SEQ ID NO: 4 by substitution, deletion, insertion, and/or addition of one (1) or several amino acid residues, the protein (n) having electron transport activity in a case where the protein (n) has been combined with a protein selected from the group consisting of (i) to (l) above;
   (o) a protein consisting of an amino acid sequence having a homology of not less than 80% with respect to the amino acid sequence represented by SEQ ID NO: 4, the protein (o) having electron transport activity in a case where the protein (o) has been combined with a protein selected from the group consisting of (i) to (l) above; and
   (p) a protein encoded by a gene consisting of a base sequence represented by SEQ ID NO: 9.
[13] The transformant according to [11] or [12], wherein
   the transporter protein is a protein selected from the group consisting of (q) to (t) below,
   (q) a protein consisting of an amino acid sequence represented by SEQ ID NO: 5;
   (r) a protein derived from the amino acid sequence of SEQ ID NO: 5 by substitution, deletion, insertion, and/or addition of one (1) or several amino acid residues, the protein (r) having transport activity;
   (s) a protein consisting of an amino acid sequence having a homology of not less than 80% with respect to the amino acid sequence represented by SEQ ID NO: 5, the protein (s) having transport activity; and
   (t) a protein encoded by a gene consisting of a base sequence represented by SEQ ID NO: 10.
[14] The transformant according to any one of [10] to [13], wherein
   the transformant is a Rhodococcus bacterium.
[15] A method for producing a β-hydroxyamino acid, the method including the step of:
   culturing a transformant according to any one of [10] to [14] in a medium including an α-amino acid or an α,α-disubstituted amino acid.
[16] The method according to [15], wherein
   a gene according to [8] which gene is included in the transformant is derived from Rhodococcus.
[17] A Rhodococcus wratislaviensis C31-06 strain with an accession number of NITE BP-02370.

### Advantageous Effects of Invention

An aspect of the present invention allows an α-amino acid or α,α-disubstituted amino acid to be converted into a β-hydroxyamino acid simply and efficiently while the aspect does not require steps such as introduction of a protecting group or deprotecting.

### Description of Embodiments

The following description will discuss an embodiment of the present invention in detail. All academic and patent documents cited in the present specification are incorporated herein by reference. Further, any numerical range expressed as "A to B" in the present specification means "not less than A and not more than B" unless otherwise stated.

The term "gene" as used herein is interchangeable with "polynucleotide", "nucleic acid", and "nucleic acid molecule", and refers to a polymer of nucleotides. The gene may be in the form of DNA (for example, cDNA or genomic DNA) or RNA (for example, mRNA). The DNA or RNA may be double-stranded or single-stranded. The single-stranded DNA or RNA may be a coding strand (sense strand) or noncoding strand (antisense strand). The gene may be synthesized chemically, or the codon usage may be changed for an increased expression of a protein to be encoded. The gene may have a codon substituted with another codon as long as those codons encode an identical amino acid.

The term "protein" is used interchangeably with "peptide" or "polypeptide". A base and amino acid are expressed herein as appropriate with a single letter or a three-letter combination defined by the IUPAC and IUB.

### <1. Monooxygenase>

An embodiment of the present invention provides a monooxygenase including at least two kinds of hetero (different) subunits (in other words, a hetero monooxygenase including two or more kinds of different subunits).

A monooxygenase is a kind of oxidase, and is an enzyme that introduces a single oxygen atom into a substrate. Conventionally known monooxygenases mostly function as a single enzyme in terms of structure. No monooxygenase has been known such as a monooxygenase including a complex (that is, hetero subunits) as for an embodiment of the present invention. Thus, the discovery of the monooxygenase as an embodiment of the present invention, which monooxygenase has the above structure, is surprising. The monooxygenase is capable of catalyzing a reaction which conventional monooxygenases are incapable of catalyzing or have low reaction activity. An embodiment of the present invention thus provides a new and useful monooxygenase that has been absent conventionally.

A monooxygenase as an embodiment of the present invention preferably includes two kinds of hetero (different) subunits. The number of subunits is not limited to any particular one as long as the subunits have monooxygenase activity even in a case where the subunits have formed a complex together with another subunit(s). The number may be, for example, two, three, four, or five.

For a monooxygenase as an embodiment of the present invention, each subunit may have any structure as long as the subunits have monooxygenase activity even in a case where the subunits have formed a complex together with another subunit(s). The subunits may each be, for example, a monomer, a dimer, a trimer, or a tetramer. A monooxygenase as an embodiment of the present invention may include, for example, a heterodimer, a heterotrimer, a heterotetramer, a heteropentamer, and/or a heterohexamer each including the above subunits, as long as the monooxygenase has monooxygenase activity.

A monooxygenase as an embodiment of the present invention has details that are changeable as appropriate as long as the monooxygenase has a characteristic structure described above. Needless to say, a monooxygenase having details that have been changed is also within the scope of the present invention.

A monooxygenase as an embodiment of the present invention is derived from a fungus such as an actinomycete. Example actinomycetes include Rhodococcus and Nocardia. The monooxygenase is derived preferably from Rhodococcus erythropolis, Rhodococcus rhodochrous, Rhodococcus wratislaviensis, or Nocardia globerula, more preferably from Rhodococcus wratislaviensis.

A monooxygenase as an embodiment of the present invention was obtained from a Rhodococcus wratislaviensis C31-06 strain as discussed later for the Examples. The Rhodococcus wratislaviensis C31-06 strain is deposited at the National Institute of Technology and Evaluation (NITE) Patent Microorganisms Depositary (#122, 2-5-8 Kazusakamatari, Kisarazu-shi, Chiba 292-0818, Japan) (depositor: Makoto Hibi, the Graduate School of Agriculture, Kyoto University [Oiwake-cho, Kitashirakawa, Sakyo-ku, Kyoto-shi, Kyoto 606-8502 Japan], accession number: NITE BP-02370 [accession date (domestic accession date): October 6, 2016; transfer date: March 12, 2018; domestic accession number before transfer: NITE P-02370]). The present invention thus also provides the Rhodococcus wratislaviensis C31-06 strain.

A monooxygenase as an embodiment of the present invention is capable of catalyzing a reaction for converting an α-amino acid or α,α-disubstituted amino acid into an optically active β-hydroxyamino acid. In other words, another preferable embodiment of the present invention is a monooxygenase capable of catalyzing a reaction for converting an α-amino acid or α,α-disubstituted amino acid into an optically active β-hydroxyamino acid, preferably a monooxygenase derived from Rhodococcus and capable of catalyzing a reaction for converting an α-amino acid or α,α-disubstituted amino acid into an optically active β-hydroxyamino acid.

The term "a-amino acid" as used herein refers to an amino acid having a monosubstituted a position. Examples of the α-amino acid for the present embodiment include, but are not limited to, L-aminobutyrate and D-aminobutyrate.

The term "α,α-disubstituted amino acid" as used herein refers to an amino acid having a disubstituted a position. Examples of the α,α-disubstituted amino acid for the present embodiment include, but are not limited to, 2-aminoisobutyric acid, L-isovaline, and D-isovaline.

The term "β-hydroxyamino acid" as used herein refers to an amino acid having a hydroxylated β position, and may be any amino acid in which a substrate such as an α-amino acid and an α,α-disubstituted amino acid has been caused by a monooxygenase as an embodiment of the present invention to have optical activity. Examples of the β-hydroxyamino acid for the present embodiment include α-methyl-D-serine, (2S,3S)-2-methyl threonine, (2R,3R)-2-methyl threonine, L-allo-threonine, and D-allo-threonine. The term "optically active β-hydroxyamino acid" as used herein is synonymous with "β-hydroxyamino acid".

A monooxygenase as an embodiment of the present invention is presumed to catalyze hydroxylation of a β position of an α-amino acid or α,α-disubstituted amino acid for conversion thereof into a β-hydroxyamino acid. The α-amino acid, α,α-disubstituted amino acid, and β-hydroxyamino acid may be any α-amino acid, α,α-disubstituted amino acid, and β-hydroxyamino acid as long as the monooxygenase converts an α-amino acid or α,α-disubstituted amino acid into an optically active β-hydroxyamino acid through the above action mechanism.

For an embodiment of the present invention, the α-amino acid or α,α-disubstituted amino acid to be converted into a β-hydroxyamino acid by a monooxygenase is preferably a compound represented by Formula (1) below. 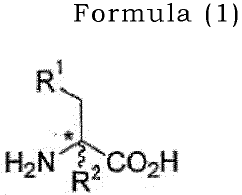 where R¹ and R² are each independently a hydrogen or alkyl group.

In a case where R¹ is an alkyl group, the alkyl group may have 1 to 5 carbon atoms, preferably 1 to 3 carbon atoms, more preferably 1 carbon atom. In a case where R¹ is an alkyl group, the alkyl group may be linear, branched, or cyclic, preferably linear.

In a case where R² is an alkyl group, the alkyl group is similar to R¹ above.

The asterisk in the formula may or may not represent an asymmetric carbon atom.

For an embodiment of the present invention, the β-hydroxyamino acid into which an α-amino acid or α,α-disubstituted amino acid is to be converted by a monooxygenase is preferably a compound represented by Formula (2) below. 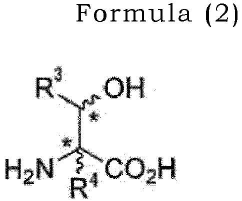 where R³ and R⁴ are each independently a hydrogen or alkyl group.

In a case where R³ is an alkyl group, the alkyl group may have 1 to 5 carbon atoms, preferably 1 to 3 carbon atoms, more preferably 1 carbon atom. In a case where R³ is an alkyl group, the alkyl group may be linear, branched, or cyclic, preferably linear.

In a case where R⁴ is an alkyl group, the alkyl group is similar to R³ above.

The asterisks in the formula may or may not represent an asymmetric carbon atom. Preferably, either of the two asterisks may be an asymmetric carbon atom. More preferably, both of the two asterisks may be asymmetric carbon atoms.

For an embodiment of the present invention, a monooxygenase preferably catalyzes at least one of the respective reactions represented by Formulae (3) to (7) below which reactions are each for converting an α-amino acid or α,α-disubstituted amino acid into an optically active β-hydroxyamino acid.

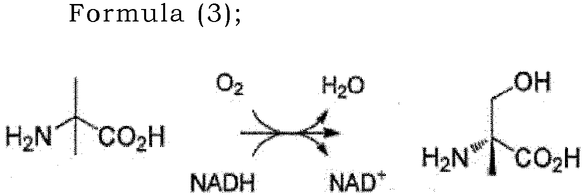

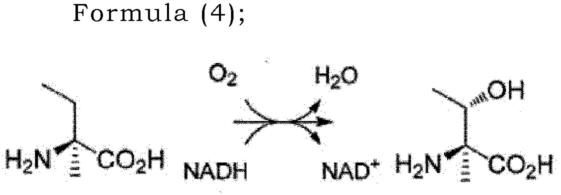

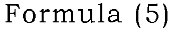

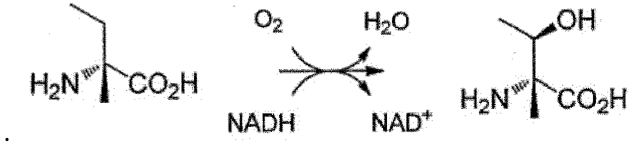

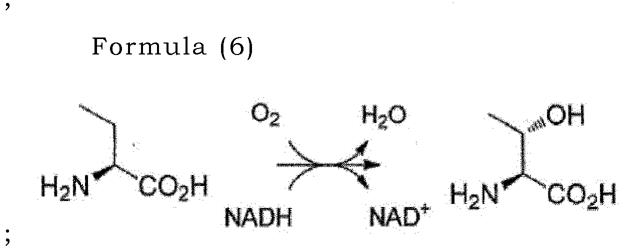

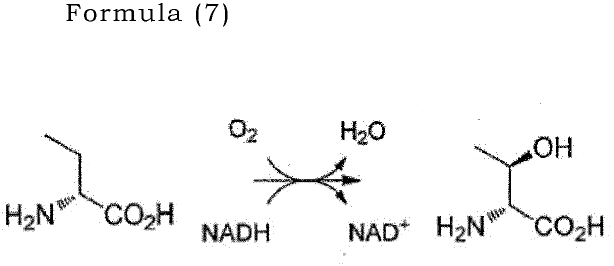

A monooxygenase as an embodiment of the present invention is preferably capable of catalyzing the at least one of the respective reactions represented by the formulae above which reactions are each for converting an α-amino acid or α,α-disubstituted amino acid into an optically active β-hydroxyamino acid in such a manner that the resulting β-hydroxyamino acid has an optical purity of not less than 60%, more preferably not less than 80%, even more preferably not less than 85%, particularly preferably not less than 87%.

A monooxygenase as an embodiment of the present invention is preferably arranged such that assuming that the two kinds of hetero subunits are an a subunit and a β subunit, the a subunit includes a protein selected from the group consisting of (a) to (d) below, and the β subunit includes a protein selected from the group consisting of (e) to (h) below,
(a) a protein consisting of an amino acid sequence represented by SEQ ID NO: 1;
(b) a protein derived from the amino acid sequence of SEQ ID NO: 1 by substitution, deletion, insertion, and/or addition of one (1) or several amino acid residues, a subunit including the protein (b) having monooxygenase activity in a case where the subunit including the protein (b) has formed a complex together with the β subunit;
(c) a protein consisting of an amino acid sequence having a homology of not less than 80% with respect to the amino acid sequence represented by SEQ ID NO: 1, a subunit including the protein (c) having monooxygenase activity in a case where the subunit including the protein (c) has formed a complex together with the β subunit;
(d) a protein encoded by a gene consisting of a base sequence represented by SEQ ID NO: 6;
(e) a protein consisting of an amino acid sequence represented by SEQ ID NO: 2;
(f) a protein derived from the amino acid sequence of SEQ ID NO: 2 by substitution, deletion, insertion, and/or addition of one (1) or several amino acid residues, a subunit including the protein (f) having monooxygenase activity in a case where the subunit including the protein (f) has formed a complex together with the a subunit;
(g) a protein consisting of an amino acid sequence having a homology of not less than 80% with respect to the amino acid sequence represented by SEQ ID NO: 2, a subunit including the protein (g) having monooxygenase activity in a case where the subunit including the protein (g) has formed a complex together with the a subunit, and
(h) a protein encoded by a gene consisting of a base sequence represented by SEQ ID NO: 7.

The protein of (a) above is a protein consisting of the amino acid sequence represented by SEQ ID NO: 1. The protein of (e) above is a protein consisting of the amino acid sequence represented by SEQ ID NO: 2. Each of the proteins is derived from Rhodococcus wratislaviensis. SEQ ID NO: 1 represents a polypeptide consisting of 378 amino acid residues in total, the polypeptide having a Protein ID of "peg.803" labeled in Protein Discover Software (available from Thermo Scientific). SEQ ID NO: 2 represents a polypeptide consisting of 373 amino acid residues in total, the polypeptide having a Protein ID of "peg.804" labeled in Protein Discover Software (available from Thermo Scientific).

The respective proteins of (b) and (f) above are each, for example, a functionally equivalent mutant, derivative, variant, allele, homolog, ortholog, or partial peptide of a protein consisting of the corresponding one of the respective amino acid sequences represented by SEQ ID NO: 1 and NO: 2 or a fusion protein produced from the above protein and another protein or peptide. The protein (b) is not limited in terms of the detailed sequence as long as a subunit including the protein (b) has monooxygenase activity in a case where the subunit including the protein (b) has formed a complex together with the β subunit. The protein (f) is not limited in terms of the detailed sequence as long as a subunit including the protein (f) has monooxygenase activity in a case where the subunit including the protein (f) has formed a complex together with the a subunit. The number of amino acids that may be deleted, substituted, or added may be any number as long as the monooxygenase maintains the above functions. The number is, however, a number of which amino acids may be deleted, substituted, or added by a publicly known introduction method such as site-directed mutagenesis. The number is preferably not more than five amino acids, more preferably not more than three amino acids (for example, three, two, or one amino acid). The term "mutation" as used herein refers mainly to a mutation introduced artificially by, for example, site-directed mutagenesis, but may also refer to a similar, naturally occurring mutation.

An amino acid residue substituted has preferably been substituted with another amino acid having an amino acid side chain of which the property is similar. Examples of the property of an amino acid side chain include a hydrophobic amino acid (A, I, L, M, F, P, W, Y, V), a hydrophilic amino acid (R, D, N, C, E, Q, G, H, K, S, T), an amino acid having an aliphatic side chain (G, A, V, L, I, P), an amino acid having a hydroxy group-containing side chain (S, T, Y), an amino acid having a sulphur atom-containing side chain (C, M), an amino acid having a carboxylic acid or an amide-containing side chain (D, N, E, Q), an amino acid having a base-containing side chain (R, K, H), and an amino acid having an aromatic side chain (H, F, Y, W) (the parentheses show single-letter expressions of amino acids). A polypeptide is known to maintain its biological activity even in a case where an amino acid sequence included in the polypeptide has been modified through deletion of one or more amino acid residues, addition of one or more amino acid residues, and/or substitution of one or more amino acid residues with another amino acid. An amino acid residue as mutated has more preferably been mutated into an amino acid residue sharing as many properties as possible with the amino acid residue before the mutation.

The expression "functionally equivalent" as used herein indicates that a protein has a biological and/or biochemical function equivalent (that is, identical or similar) to that of the protein of interest. Biological properties include, for example, specificity of a site of expression and the amount of expression. Whether a protein into which a mutation has been introduced has a desired function may be determined by studying whether using that protein as part of a subunit allows the complex to have monooxygenase activity.

The respective proteins of (c) and (g) above are also each intended to be, for example, a functionally equivalent mutant, derivative, variant, allele, homolog, ortholog, or partial peptide of a protein consisting of the corresponding one of the respective amino acid sequences represented by SEQ ID NO: 1 and NO: 2 or a fusion protein produced from the above protein and another protein or peptide. The protein (c) is not limited in terms of the detailed sequence as long as a subunit including the protein (c) has monooxygenase activity in a case where the subunit including the protein (c) has formed a complex together with the β subunit. The protein (g) is not limited in terms of the detailed sequence as long as a subunit including the protein (g) has monooxygenase activity in a case where the subunit including the protein (g) has formed a complex together with the a subunit.

An amino acid sequence having a homology means that the amino acid sequence has a sequence identity of at least not less than 80%, preferably not less than 85%, more preferably not less than 90%, even more preferably not less than 95% (for example, not less than 95%, 96%, 97%, 98%, or 99%), with respect to the entire amino acid sequence (or a region necessary for functional expression). The homology of an amino acid sequence may be determined with use of a program for BLASTN (nucleic acid level) or BLASTX (amino acid level) (Altschul et al. J. Mol. Biol., 215: 403-410, 1990). This program is based on the algorithm BLAST by Karlin and Altschul (Proc. Natl. Acad. Sci. USA, 87:2264-2268, 1990, Proc. Natl. Acad. Sci. USA, 90: 5873-5877, 1993). In a case where BLASTN is used to analyze a base sequence, the parameters are set as follows, for example: score = 100, wordlength = 12. In a case where BLASTX is used to analyze an amino acid sequence, the parameters are set as follows, for example: score = 50, wordlength = 3. In a case where the Gapped BLAST program is used to analyze an amino acid sequence, the analysis may be carried out as discussed in Altschul et al. (Nucleic Acids Res. 25: 3389-3402, 1997). In a case where BLAST and the Gapped BLAST program are used, the default parameters of each program are used. Specifically how analysis is carried out on the basis of each of the above approaches is publicly known. Addition or deletion (for example, a gap) may be accepted so that a base sequence or amino acid sequence to be compared is aligned in an optimal state.

The term "homology" as used herein refers to the proportion (for example, positive) of the number of amino acid residues sharing a similar property, but more preferably refers to identity, that is, the proportion of the number of identical amino acid residues. The properties of an amino acid are as described above.

For the protein of (d) above, SEQ ID NO: 6 represents a base sequence (open reading frame or ORF) of a gene encoding a protein consisting of the amino acid sequence represented by SEQ ID NO: 1. For the protein of (h) above, SEQ ID NO: 7 represents a base sequence (ORF) of a gene encoding a protein consisting of the amino acid sequence represented by SEQ ID NO: 2.

The above gene and proteins may each be obtained by a typical method for modifying a polynucleotide. Specifically, substituting, deleting, inserting, and/or adding particular bases of a polynucleotide which bases have genetic information of the protein allows a polynucleotide to be prepared that has genetic information of a desired recombinant protein. Specific examples of a method for converting bases of a polynucleotide include using a commercially available kit (for example, KOD-Plus Site-Directed Mutagenesis Kit [available from Toyobo Co., Ltd.], Transformer Site-Directed Mutagenesis Kit [available from Clontech], or QuickChange Site Directed Mutagenesis Kit [available from Stratagene]) and using polymerase chain reaction. These methods are known to persons skilled in the art.

The gene may consist only of a polynucleotide encoding the protein or may include another base sequence added thereto. A base sequence to be added is not limited to any particular one. Examples of the base sequence to be added include a base sequence encoding a label (for example, histidine tag, Myc tag, or FLAG tag), a fusion protein (for example, streptavidin, cytochrome, GST, GFP, or MBP), a promoter sequence, and a signal sequence (for example, endoplasmic reticulum localization signal sequence or secretion sequence). The site to which such a base sequence is to be added is not limited to any particular one. The site may be, for example, the N-terminus or C-terminus of a protein to be translated.

### <2. Monooxygenase reaction system>

An embodiment of the present invention provides a monooxygenase reaction system for producing a β-hydroxyamino acid, the system including the monooxygenase and an electron transport chain protein.

The monooxygenase reaction system is a system that involves the monooxygenase producing a β-hydroxyamino acid with use of oxidation-reduction power provided by means of an electron transport chain protein. The monooxygenase reaction system is not limited to any particular one as long as monooxygenase reaction system includes the monooxygenase and an electron transport chain protein and produces a β-hydroxyamino acid.

The monooxygenase reaction system may be a system developed inside the body of an organism or a system developed artificially outside the body of an organism. The monooxygenase reaction system is, in view of stability and activity of a monooxygenase protein, preferably a system developed inside the body of an organism.

The development of the monooxygenase reaction system inside the body of an organism may be such that the organism has the system inherently or that the system is artificially introduced into an organism that does not have the system.

The monooxygenase reaction system may be artificially introduced into any target by any method. The monooxygenase reaction system may be introduced by, for example, a method described under <4. Vector> and <5. Transformant> below.

The electron transport chain protein for the monooxygenase reaction system is not limited to any particular one as long as the protein provides oxidation-reduction power for allowing the monooxygenase to have monooxygenase activity.

Examples of the electron transport chain protein include a combination of ferredoxin (for example, a Rieske protein, putidaredoxin, or adrenodoxin) and ferredoxin reductase (for example, flavoprotein reductase, putidaredoxin reductase, or adrenodoxin reductase), a combination of flavodoxin and flavodoxin reductase, and P450 reductase.

An embodiment of the present invention is preferably arranged such that the electron transport chain protein includes a protein selected from the group consisting of (i) to (l) below and a protein selected from the group consisting of (m) to (p) below,
(i) a protein consisting of an amino acid sequence represented by SEQ ID NO: 3;
(j) a protein derived from the amino acid sequence of SEQ ID NO: 3 by substitution, deletion, insertion, and/or addition of one (1) or several amino acid residues, the protein (j) having electron transport activity in a case where the protein (j) has been combined with a protein selected from the group consisting of (m) to (p) below;
(k) a protein consisting of an amino acid sequence having a homology of not less than 80% with respect to the amino acid sequence represented by SEQ ID NO: 3, the protein (k) having electron transport activity in a case where the protein (k) has been combined with a protein selected from the group consisting of (m) to (p) below;
(l) a protein encoded by a gene consisting of a base sequence represented by SEQ ID NO: 8;
(m) a protein consisting of an amino acid sequence represented by SEQ ID NO: 4;
(n) a protein derived from the amino acid sequence of SEQ ID NO: 4 by substitution, deletion, insertion, and/or addition of one (1) or several amino acid residues, the protein (n) having electron transport activity in a case where the protein (n) has been combined with a protein selected from the group consisting of (i) to (l) above;
(o) a protein consisting of an amino acid sequence having a homology of not less than 80% with respect to the amino acid sequence represented by SEQ ID NO: 4, the protein (o) having electron transport activity in a case where the protein (o) has been combined with a protein selected from the group consisting of (i) to (l) above; and
(p) a protein encoded by a gene consisting of a base sequence represented by SEQ ID NO: 9.

The protein of (i) above is a protein consisting of the amino acid sequence represented by SEQ ID NO: 3. The protein of (m) above is a protein consisting of the amino acid sequence represented by SEQ ID NO: 4. Each of the proteins is derived from Rhodococcus wratislaviensis. SEQ ID NO: 3 represents a polypeptide consisting of 322 amino acid residues in total, the polypeptide having a Protein ID of "peg.801" labeled in Protein Discover Software (available from Thermo Scientific). SEQ ID NO: 4 represents a polypeptide consisting of 169 amino acid residues in total, the polypeptide having a Protein ID of "peg.802" labeled in Protein Discover Software (available from Thermo Scientific).

The respective proteins of (j) and (n) above are each, for example, a functionally equivalent mutant, derivative, variant, allele, homolog, ortholog, or partial peptide of a protein consisting of the corresponding one of the respective amino acid sequences represented by SEQ ID NO: 3 and NO: 4 or a fusion protein produced from the above protein and another protein or peptide. The protein (j) is not limited in terms of the detailed sequence as long as it has electron transport activity in a case where the protein (j) has been combined with a protein selected from the group consisting of (m) to (p). The protein (n) is not limited in terms of the detailed sequence as long as it has electron transport activity in a case where the protein (n) has been combined with a protein selected from the group consisting of (i) to (l).

The respective proteins of (k) and (o) above are also each intended to be, for example, a functionally equivalent mutant, derivative, variant, allele, homolog, ortholog, or partial peptide of a protein consisting of the corresponding one of the respective amino acid sequences represented by SEQ ID NO: 3 and NO: 4 or a fusion protein produced from the above protein and another protein or peptide. The protein (k) is not limited in terms of the detailed sequence as long as it has electron transport activity in a case where the protein (k) has been combined with a protein selected from the group consisting of (m) to (p). The protein (o) is not limited in terms of the detailed sequence as long as it has electron transport activity in a case where the protein (o) has been combined with a protein selected from the group consisting of (i) to (l).

For the protein of (l) above, SEQ ID NO: 8 represents a base sequence (open reading frame or ORF) of a gene encoding a protein consisting of the amino acid sequence represented by SEQ ID NO: 3. For the protein of (p) above, SEQ ID NO: 9 represents a base sequence (ORF) of a gene encoding a protein consisting of the amino acid sequence represented by SEQ ID NO: 4.

For the present embodiment, the electron transport chain protein is not limited to a protein selected from the group consisting of (i) to (l) and a protein selected from the group consisting of (m) to (p), and may be another protein involved with the electron transport system.

For general descriptions of a gene and proteins (for example, term definitions), reference is made to the descriptions under <1. Monooxygenase>.

### <3. Gene>

An embodiment of the present invention provides a monooxygenase gene encoding one of the above proteins.

For the present embodiment, the proteins may be those of which the monooxygenase consists as an embodiment of the present invention described under <1. Monooxygenase>.

### <4. Vector>

An embodiment of the present invention provides a vector including the gene described under <3. Gene>. The vector may be an expression vector for expressing the above gene in a host cell to prepare a transformant or a vector for use in producing a recombinant protein.

A base vector as a matrix of the vector may be any of various vectors in common use. The base vector may be, for example, a plasmid, a phage, or a cosmid. An appropriate base vector may be selected according to (i) the cell into which the vector is to be introduced or (ii) the method by which the vector is to be introduced. In other words, the specific kind of vector is not limited to any particular one, and a vector that can be expressed in a host cell is selected as appropriate. A promoter sequence is selected as appropriate according to the kind of host cell to ensure the expression of the gene, and is incorporated in, for example, various plasmids together with the gene for use as an expression vector. Examples of the expression vector include a phage vector, a plasmid vector, a viral vector, a retroviral vector, a chromosomal vector, an episomal vector, and a virus-derived vector (for example, a bacterial plasmid, a bacteriophage, a yeast episome, a yeast chromosome element, and a virus [for example, baculovirus, papovavirus, vaccinia virus, adenovirus, avipoxvirus, pseudorabies virus, herpesvirus, lentivirus, and retrovirus]) as well as a vector derived from a combination thereof (for example, a cosmid and a phagemid).

Example vectors that can be used preferably for bacteria include pQE60, pQE70, pQE80, and pQE9 (available from Qiagen); pTipQC1 (available from Qiagen or Hokkaido System Science) and pTipRT2 (available from Hokkaido System Science); pBS vector, Phagescript vector, Bluescript vector, pNH8A, pNH16A, pNH18A, and pNH46A (available from Stratagene); ptrc99a, pKK223-3, pKK233-3, pDR540, and pRIT5 (available from Addgene); pRSF (available from MERCK); and pAC (available from NIPPON GENE). Examples of preferable eukaryote vectors include pWLNE0, pSV2CAT, pOG44, pXT1, and pSG (available from Stratagene); and pSVK3, pBPV, pMSG, and pSVL (available from Addgene).

An insert of the gene is preferably linked operably with an appropriate promoter. Other appropriate promoters are promoters known to persons skilled in the art, and are not limited to any particular ones. Examples include lacUV5 promotor, trp promotor, trc promotor, tac promotor, lpp promotor, tufB promotor, recA promotor, pL promotor, lacI promotor, lacZ promotor, T3 promotor, T7 promotor, SV40 early and late promoters, and a promoter of retrovirus LTR.

The vector preferably further includes a site for transcription initiation, a site for transcription termination, and a transcriptional region including a ribosome-binding site for translation. A matured transcript expressed by a vector construct includes a coding region including (i) transcription initiation AUG at the start of a polypeptide to be translated and (ii) a stop codon appropriately positioned at the end of the polypeptide.

A host into which the vector is to be introduced is not limited to any particular one, and may suitably be any of various cells. Typical examples of an appropriate host include, but are not limited to, bacteria, yeast, filamentous fungi, plant cells, and animal cells. The host cell is cultured in an appropriate medium publicly known in the art and under an appropriate condition publicly known in the art.

The method by which the vector is to be introduced into a host cell (that is, the transformation method) is not limited to any particular one. The method may suitably be a conventionally publicly known one such as calcium phosphate method, liposome method, DEAE-dextran method, microinjection, cationic lipid-mediated transfection, electroporation, transduction, and infection. These methods are discussed in many standard laboratory manuals such as Davis et al., Basic Methods In Molecular Biology (1986).

### <5. Transformant>

An embodiment of the present invention provides a transformant including the gene described under <3. Gene> or the recombinant vector described under <4. Vector>. The expression "including a gene or a vector" indicates that the gene or vector has been introduced into a target cell (host cell) by a publicly known genetic engineering approach so that the gene or vector can be expressed. The term "transformant" covers not only a cell, tissue, and organ, but also an individual organism.

The transformant may be prepared (produced) by, for example, a method of transforming the above vector. An organism as a target of the transformation is not limited to any particular one, and may be, for example, any of various organisms listed as examples for the host cell.

Examples of a host cell for use in an embodiment of the present invention include bacteria, yeast, filamentous fungi, plant cells, and animal cells. The host cell is preferably an actinomycete in terms of introduction and expression efficiency.

Example actinomycetes include Rhodococcus and Nocardia bacteria. The actinomycete is preferably, for example, Rhodococcus erythropolis, Rhodococcus rhodochrous, Rhodococcus wratislaviensis, or Nocardia globerula. The host cell is particularly preferably Rhodococcus erythropolis as in the Examples below.

For an embodiment of the present invention, the transformant preferably further includes:
(1) a gene encoding an electron transport chain protein; and/or
(2) a gene encoding a transporter protein.

The electron transport chain protein is not limited to any particular one. Examples include the electron transport chain protein described under <2. Monooxygenase reaction system>.

The transporter protein may be any protein having the function of uptake, into the transformant, of a substance that is to serve as a substrate of the monooxygenase.

Examples of the transporter protein include a permease (for example, a symporter, an antiporter, or a uniporter) and an ABC transporter.

For an embodiment of the present invention, the transporter protein is preferably a protein selected from the group consisting of (q) to (t) below,
(q) a protein consisting of an amino acid sequence represented by SEQ ID NO: 5;
(r) a protein derived from the amino acid sequence of SEQ ID NO: 5 by substitution, deletion, insertion, and/or addition of one (1) or several amino acid residues, the protein (r) having transport activity;
(s) a protein consisting of an amino acid sequence having a homology of not less than 80% with respect to the amino acid sequence represented by SEQ ID NO: 5, the protein (s) having transport activity; and
(t) a protein encoded by a gene consisting of a base sequence represented by SEQ ID NO: 10.

The protein of (q) above is a protein consisting of the amino acid sequence represented by SEQ ID NO: 5, and is derived from Rhodococcus wratislaviensis. SEQ ID NO: 5 represents a polypeptide consisting of 480 amino acid residues in total, the polypeptide having a Protein ID of "peg.800" labeled in Protein Discover Software (available from Thermo Scientific).

The protein of (r) above is, for example, a functionally equivalent mutant, derivative, variant, allele, homolog, ortholog, or partial peptide of a protein consisting of the amino acid sequence represented by SEQ ID NO: 5 or a fusion protein produced from the above protein and another protein or peptide. The protein of (r) is not limited in terms of the detailed sequence as long as it has transport activity.

The protein of (s) above is also intended to be, for example, a functionally equivalent mutant, derivative, variant, allele, homolog, ortholog, or partial peptide of a protein consisting of the amino acid sequence represented by SEQ ID NO: 5 or a fusion protein produced from the above protein and another protein or peptide. The protein of (s) is not limited in terms of the detailed sequence as long as it has transport activity.

For the protein of (t) above, SEQ ID NO: 10 represents a base sequence (open reading frame or ORF) of a gene encoding a protein consisting of the amino acid sequence represented by SEQ ID NO: 5.

For the present embodiment, the transporter protein is not limited to a protein selected from the group consisting of (q) to (t) above, and may be another protein involved with substance transport on a cell membrane.

For general descriptions of a gene and proteins (for example, term definitions), reference is made to the descriptions under <1. Monooxygenase>.

The transformant including (1) a gene encoding an electron transport chain protein promotes provision of oxidation-reduction power for the monooxygenase and thereby advantageously allows a β-hydroxyamino acid to be produced efficiently.

The above (1) a gene encoding an electron transport chain protein functions to, in a case where a host/transformant into which the gene is to be introduced does not have an electron transport chain protein for providing oxidation-reduction power for the monooxygenase, compensate for the lack of an electron transport chain protein and in a case where a host/transformant into which the gene is to be introduced does have an electron transport chain protein for providing oxidation-reduction power for the monooxygenase, enhance the provision of oxidation-reduction power.

The transformant including (2) a gene encoding a transporter protein promotes uptake of a monooxygenase substrate into the transformant and thereby advantageously allows a β-hydroxyamino acid to be produced efficiently.

### <6. Method for producing β-hydroxyamino acid>

An embodiment of the present invention provides a method for producing a β-hydroxyamino acid, the method including the step of culturing the transformant described under <5. Transformant> in a medium including an α-amino acid or α,α-disubstituted amino acid. The production of a β-hydroxyamino acid according to the present embodiment is production involving use of the transformant described under <5. Transformant>, and is not particularly limited in terms of, for example, other specific arrangements, conditions, materials, or equipment for use.

The transformant is preferably an actinomycete, in particular Rhodococcus bacteria. The transformant is preferably Rhodococcus erythropolis among others. Using such bacteria allows a β-hydroxyamino acid to be produced highly efficiently.

For the present embodiment, the production of a β-hydroxyamino acid with use of the transformant is achieved by the presence, in a medium, of both the transformant and an α-amino acid or α,α-disubstituted amino acid that is to serve as a substrate. When to add an α-amino acid or α,α-disubstituted amino acid to a medium is not limited to any particular time. An α-amino acid or α,α-disubstituted amino acid may be added, for example, at the same time as the expression of an introduced gene in the transformant or either or both of before and after the expression of an introduced gene in the transformant. The present embodiment may be arranged such that an α-amino acid or α,α-disubstituted amino acid is added to a medium already including the transformant or that the transformant is put into a medium already including an α-amino acid or α,α-disubstituted amino acid. The conditions are not limited as above, and may be set as appropriate by a person skilled in the art so that a β-hydroxyamino acid can be produced efficiently.

For the present embodiment, the α-amino acid, the α,α-disubstituted amino acid, and the β-hydroxyamino acid may be those described under <1. Monooxygenase>.

Regarding the step of culturing the transformant, a method for culturing a host strain for the transformant may suitably be a conventionally publicly known one, and is not limited to any particular one. The temperature during the culture is, for example, preferably 20°C to 45°C, more preferably 25°C to 35°C, as with a usual method. The reaction time is also not limited to any particular length. The culture is preferably 1 hour to 120 hours long, more preferably 24 hours to 72 hours long, from the expression of an introduced gene.

The method for collecting a β-hydroxyamino acid from a culture or bacterial cell may be a method normally used to obtain a microorganism product, and is not limited to any particular one.

The descriptions of items <1> to <6> above are applicable as appropriate to other items as well. The present invention is not limited to the embodiments, but can be altered by a skilled person in the art within the scope of the claims. The present invention also encompasses, in its technical scope, any embodiment derived by combining technical means disclosed in differing embodiments. The following description will discuss the present invention on the basis of Examples. The present invention is, however, not limited to the Examples only.

### Examples

### [Example 1] Isolation of 2-aminoisobutyric acid-utilizing microorganism and evaluation of hydroxylation activity

With use of 2 ml of a liquid medium for accumulation which liquid medium included 0.1% 2-aminoisobutyric acid, 0.1% ammonium chloride, 0.1% potassium dihydrogenphosphate, 0.1% dipotassium hydrogenphosphate, 0.03% magnesium sulfate heptahydrate, and 0.01% Difco Yeast Nitogen Base w/o Amino Acids and Ammoniumu Sulfate, various samples of soil collected from the natural world were shake-cultured at 28°C for 5 days (the concentration is expressed in (w/v)% [the same applies throughout the Examples]). A culture solution in which a microorganism was starting to grow was inoculated into 2 ml of fresh liquid medium for accumulation. After this operation was carried out several times, a 2-aminoisobutyric acid-utilizing microorganism was isolated from a 1.5% agar plate medium prepared with 1.5% agar plate medium containing the same components as the above liquid medium. Each isolated microorganism was inoculated again in 2 ml of a liquid medium for accumulation, and was shake-cultured at 28°C for 5 days. Then, a centrifugal operation was carried out at 8000 g for 10 minutes to collect bacteria. The bacteria were washed twice with a 0.85% saline solution. The wet cell was used for a resting microorganism reaction below. The resting microorganism reaction was carried out by shaking the mixture of 10 mM of 2-aminoisobutyric acid, 10 mM of glucose, 1 mM of aminooxyacetic acid, and 5% wet cell in 50 mM of HEPES buffer (pH 7.5) at 300 rpm for 4 hours.

The results are shown in Table 1. Table 1 indicates production of α-methyl-D-serine in three kinds of microorganisms.

**[Table 1]**

| Strain | Accession number for international deposit | Species | Specific activity¹ | % e.e.² |
|---|---|---|---|---|
| C31-06 | NITE BP-02370 | *Rhodococcus wratislaviensis* | 0.14 | 88.2 |
| C63-06 | | *Rhodococcus wratislaviensis* | 0.053 | 85.4 |
| C86-07 | | *Nocardia globerula* | 0.24 | 67.9 |

| | | | | |
|---|---|---|---|---|
| ¹ 1 nmol of MeSer per min per mg of wet cell ² Enantiomeric excess of α-methyl-D-serine | | | | |

### <Analysis conditions>

- Measurement device: LCMS-2010A (Shimadzu Corporation)
- Column: Xbridge C18 column (5 µm: 2.1 × 150 mm) (Nihon Waters K.K.)
- Column oven temperature: 40°C
- Mobile phase A: 10 mM of ammonium acetate (pH 5.0)
- Mobile phase B: methanol
- Flow rate: 0.3 ml/min
- Gradient setting: 0 to 0.5 min, 0 to 1% mobile phase B; 0.5 to 18 min, 1 to 5% mobile phase B; 18 to 19 min, 5 to 9% mobile phase B; 19 to 29.5 min, 9 to 17% mobile phase B; 29.5 to 40 min, 17 to 60% mobile phase B
- MS condition: block temperature: 200°C, Curved desolvation line temperature: 250°C, Detector voltage: 1.5 kV, nebulizing gas flow: 1.51/min

An amino acid in an analysis solution was derivatized with use of AccQ-Tag derivation kit (Nihon Waters K.K.), and was then subjected to LCMS analysis.

### [Example 2] Proteome analysis of R. wratislaviensis C31-06 strain

R. wratislaviensis C31-06 strains were each cultured at 28°C for 38.5 hours with use of 250 ml of 2-aminoisobutyric acid-induced liquid medium (0.1% 2-aminoisobutyric acid, 0.1% ammonium chloride, 0.1% potassium dihydrogenphosphate, 0.1% dipotassium hydrogenphosphate, 0.03% magnesium sulfate heptahydrate, and 0.01% Difco Yeast Nitogen Base w/o Amino Acids and Ammoniumu Sulfate) or 250 ml of a non-induced medium (which was identical to a 2-aminoisobutyric acid-induced liquid medium except that 0.1% 2-aminoisobutyric acid was replaced with 0.05% glucose) (for each, n = 3). Each cultured bacterial cell was collected through centrifugation at 4°C and 8000 g for 10 minutes, and was suspended in 50 mM of a Tris-HCl (pH8.0) buffer including 7M of urea, 2.0 mM of thiourea, 2% CHAPS, 10 mM of dithiothreitol, and 1 tablet / 10 ml of protease inhibitor (Complete Mini, Roche). Then, 0.10-mm beads were added. The bacterial cell was crushed with use of Multi Beads Shocker. Then, the bacterial cell was centrifuged at 4°C and 20000 g for 15 minutes. The centrifugal supernatant obtained was used as a cell lysate. The precipitate was washed with a minimum amount of 200 mM of TEAB buffer (pH 8.0). The supernatant and the cell lysate together were filtered through a 0.45 µm filter. Next, the filtrate was substituted with 200 mM TEAB buffer. Then, 9.5 mM of tris(2-carboxyethyl)phosphine was added to the substituted filtrate for treatment at 55°C for 60 minutes. After that, iodoacetamide was added in an amount of 17.9 mM for treatment at room temperature for 30 minutes. Finally, cold acetone was added in an amount 2 to 4 times larger for treatment at -20°C for 3 hours. The resulting product was centrifuged at 4°C and 13000 g for 10 minutes for collection of protein. The remaining acetone was removed through treatment at 37°C for 2 minutes.

Next, tryptic digestion of the protein was carried out. Specifically, the collected protein was suspended in 200 mM of TEAB buffer, and was then digested with use of 47.6 ng/µl of trypsin at 37°C overnight. After that, the resulting digest was labeled in 41 µl of acetonitrile with use of tandem mass tag (TMT) 6-plex labeling kit (Thermo Fisher Scientific). The digest reacted at room temperature for 60 minutes. Then, 8 µl of 5% hydroxylamine was added, and was mixed for 15 minutes. Next, the liquid was evaporated in a vacuum. Then, the remainder was dissolved in 100 µl of 0.1% trifluoroacetic acid.

The resulting protein having undergone tryptic digestion was subjected to LCMS analysis (Prominence Nano Flow System [Shimadzu Corporation]). The induced protein was identified with use of the mass spectrometry data obtained and Protein Discover Software (Thermo Scientific) including a protein database prepared on the basis of genomic information for the R. wratislaviensis C31-06 strain (obtained from Hokkaido System Science). The results are shown in Table 2.

**[Table 2]**

| Protein ID | logFC | Annotated function |
|---|---|---|
| peg.3201 | 3.88 | hypothetical protein |
| peg.812 | 3.64 | 3-hydroxyisobutyrate dehydrogenase (EC 1.1.1.31) |
| peg.800 | 3.2 | D-serine/D-alanine/glycine transporter |
| peg.1024 | 3.05 | FIG00821579: hypothetical protein |
| peg.3348 | 2.99 | CAIB/BAIF family protein |
| peg.795 | 2.98 | Alanine racemase (EC 5.1.1.1) |
| peg.2135 | 2.83 | Isochorismatase (EC 3.3.2.1) of siderophore biosynthesis |
| peg.2853 | 2.8 | FIG00995544: hypothetical protein |
| peg.8283 | 2.65 | 2,3-dihydro-2,3-dihydroxybenzoate dehydrogenase (EC 1.3.1.28) |
| peg.7342 | 2.63 | OsmC family protein |
| peg.5085 | 2.58 | Pyruvate dehydrogenase E1 component beta subunit (EC 1.2.4.1) |
| peg.803 | 2.57 | b117611; hypothetical protein |
| peg.4183 | 2.56 | Iron utilization protein |
| peg.8589 | 2.44 | Ferric enterobactin-binding periplasmic protein FepB (TC 3.A.1.14.2) |
| peg.807 | 2.35 | hypothetical protein |
| peg.798 | 2.32 | Alanine dehydrogenase (EC 1.4.1.1) |
| peg.806 | 2.31 | Aldehyde dehydrogenase (EC 1.2.1.3) |
| peg.3349 | 2.28 | Aldehyde dehydrogenase (EC 1.2.1.3) |
| peg.804 | 2.24 | bII7611; hypothetical protein |
| peg.799 | 2.2 | Serine hydroxymethyltransferase (EC 2.1.2.1) |
| peg.801 | 2.18 | Flavodoxin reductases (ferredoxin-NADPH reductases) family 1 |
| peg.8070 | 2.09 | 18 kDa antigen 2 |
| peg.802 | 2.09 | rieske [2Fe-2S] domain protein |
| peg.4813 | 2.04 | peptide monooxygenase |

| | | |
|---|---|---|
| logFC was calculated by log2(induced area protein amount - non-induced area protein amount) | | |

### <Analysis conditions>

· Measurement device: Prominence Nano Flow System (Shimadzu Corporation)
· Column: Monolithic silica capillary column (500 cm long, 0.1 mm ID) (Kyoto Monotech)
· Column oven temperature: 40°C
· Mobile phase A: 0.1% aqueous formic acid solution
· Mobile phase B: 0.1% formic acid in acetonitrile
· Flow rate: 500 ml/min
· Gradient setting: 0 to 600 min, 5 to 45% mobile phase B
· MS condition: LTQ Velos linear ion trap mass spectrometer (Thermo Scientific), 2.3 kV of ESI voltage, temperature of ion transfer tube on LTQ Velos ion trap: 280°C

### [Example 3] Identification of 2-aminoisobutyric acid hydroxylase and electron transport chain protein

The proteins obtained in Example 2 and having Protein ID peg.801-804 were each expected to be a complex for catalyzing a 2-aminoisobutyric acid hydroxylation reaction. Thus, a gene encoding the protein was obtained as below.

Specifically, PCR was carried out under the PCR conditions below to obtain PCR products for peg.801-804.

### <PCR conditions>

· Template: genome of R. wratislaviensis C31-06 strain (100 ng/µl, 1 µl)
· Polymerase: PrimeSTAR Max Premix (2×) (Takara Bio Inc.) (25 µl)
· Primer (pTipQC1_5' Hyd): 
TGTTTAACTTTAAGAAGGAGATATACCATGGTTGCACCAACCTC
GAA (SEQ ID NO: 11) (10 μM, 1 μl)
· Primer (pTipQC1_3' Hyd): 
TGGTGATGGTGATGCTCGAGAGATCTACTAGAGATCGAGGACG

AGCC (SEQ ID NO: 12) (10 μM, 1 μl)
· Amplification condition: 30 cycles of 98°C, 10 seconds; 55°C, 5 seconds; and 72°C, 1 minute

The PCR product was inserted, with use of NEBuilder HiFi DNA Assembly Master Mix (New England BioLabs), into a vector pTipQC1 (Hokkaido System Science) treated with restriction enzymes NcoI and HindIII. Next, E.coli JM109 was transformed with use of the plasmid pQAH1. The transformant was cultured at 28°C overnight in 2 ml of LB medium (including 50 µg/ml of ampicillin). pQAH1 was obtained from the bacterial cell, and then Rhodococcus erythropolis L88 (Hokkaido System Science) was transformed with use of the plasmid. The transformant was cultured at 28°C overnight in 2 ml of LB medium (including 20 µg/ml of chloramphenicol). Part of the culture solution was added to 2 ml of fresh LB medium (including 20 µg/ml of chloramphenicol), and was shake-cultured at 28°C. After the turbidity of the culture solution reached 0.8 (absorption wavelength: 600 nm), thiostrepton was added at a concentration of 0.2 µg/ml for the purpose of inducing expression of a protein. Next, 2-aminoisobutyric acid was added at a concentration of 10 mM as a reactive substrate. Shaking reaction was then started.

As a result, after 48 hours of reaction, production of 2.7 mM of α-methyl-D-serine was determined.

The above operation showed that peg.801-804 formed a complex for catalyzing a 2-aminoisobutyric acid hydroxylation reaction.

### <Analysis conditions>

· Measurement device: Shimadzu LC-VP (Shimadzu Corporation)
· Column: Xbridge C18 column (5 µm: 2.1 × 150 mm) (Nihon Waters K.K.)
· Column oven temperature: 40°C
· Mobile phase A: Waters AccQ-Tag Eluent A
· Mobile phase B: methanol
· Flow rate: 0.3 ml/min
· Gradient setting: 0 to 0.1 min, 0% mobile phase B; 0.1 to 0.5 min, 0 to 1% mobile phase B; 0.5 to 18 min, 1 to 5% mobile phase B; 18 to 19 min, 5 to 9% mobile phase B; 19 to 29.5 min, 9 to 17% mobile phase B; 29.5 to 40 min, 17 to 60% mobile phase B; 40 to 43 min, 60 to 0% mobile phase B; 43 to 55 min, 0% mobile phase B
· Detection: Fluorescence detector (excitation wavelength: 250 nm, emission wavelength: 395 nm)

An amino acid in an analysis solution was derivatized with use of AccQ-Tag derivation kit (Nihon Waters K.K.), and was then subjected to LC analysis.

### [Example 4] Function analysis of peg.803, 804

### [4.1]

A transformed actinomycete that expresses the proteins with Protein ID peg.801, 802, 803 was developed as below.

Specifically, PCR was carried out under the PCR conditions below to obtain PCR products for peg.801, 802, 803.

### <PCR conditions>

· Template: genome of R. wratislaviensis C31-06 strain (100 ng/µl, 1 µl)
· Polymerase: PrimeSTAR Max Premix (2×) (Takara Bio Inc.) (25 µl)
· Primer (pTipQC1_5' 803): 
TGTTTAACTTTAAGAAGGAGATATACCATGACCATCATCGAACA
CGG (SEQ ID NO: 13) (10 μM, 1 μl)
· Primer (pTipQC1_3' Hyd): 
TGGTGATGGTGATGCTCGAGAGATCTACTAGAGATCGAGGACG
AGCC (SEQ ID NO: 12) (10 μM, 1 μl)
· Amplification condition: 30 cycles of 98°C, 10 seconds; 55°C, 5 seconds; and 72°C, 1 minute

The PCR product was inserted, with use of NEBuilder HiFi DNA Assembly Master Mix (New England BioLabs), into a vector pTipQC1 (Qiagen) treated with restriction enzymes NcoI and HindIII. Next, E.coli JM109 was transformed with use of the plasmid pQAH-d804. The transformant was cultured at 28°C overnight in 2 ml of LB medium (including 50 µg/ml of ampicillin). pQAH-d804 was obtained from the bacterial cell, and then Rhodococcus erythropolis L88 (Hokkaido System Science) was transformed with use of the plasmid. The transformant was cultured at 28°C overnight in 2 ml of LB medium (including 20 µg/ml of chloramphenicol). Part of the culture solution was added to 2 ml of fresh LB medium (including 20 µg/ml of chloramphenicol), and was shake-cultured at 28°C. After the turbidity of the culture solution reached 0.8 (absorption wavelength: 600 nm), thiostrepton was added at a concentration of 0.2 µg/ml for the purpose of inducing expression of a protein. Next, 2-aminoisobutyric acid was added at a concentration of 10 mM as a reactive substrate. Shaking reaction was then started.

As a result, after 48 hours of reaction, production of α-methyl-D-serine was not determined in the culture supernatant.

The results of Example 3 and the above results show that hydroxylation activity expression requires the peg.804 protein.

### [4.2]

Subsequently, a transformed actinomycete that expresses the proteins with Protein ID peg.801, 802, 804 was developed as below.

Specifically, PCR was carried out under the PCR conditions below to obtain PCR products for peg.801, 802, 804.

### <PCR conditions>

· Template: genome of R. wratislaviensis C31-06 strain (100 ng/µl, 1 µl)
· Polymerase: PrimeSTAR Max Premix (2×) (Takara Bio Inc.) (25 µl)
· Primer (pTipQC1_5' Hyd): 
TGTTTAACTTTAAGAAGGAGATATACCATGGTTGCACCAACCTC
GAA (SEQ ID NO: 11) (10 μM, 1 μl)
· Primer (joint802-4_3'): 
CGCTACCGATTACAAACTTGGACATTCTTAACCAACCTTTCCTG

GGC (SEQ ID NO: 14) (10 μM, 1 μl)
 or
· Primer (joint804-2_5'): CCCGAGCCCAGGAAAGGTTGGTTAAGAATGTCCAAGTTTGTAAT CGG (SEQ ID NO: 15) (10 μM, 1 μl)
· Primer (pTipQC1_3' Hyd): 
TGGTGATGGTGATGCTCGAGAGATCTACTAGAGATCGAGGACG

AGCC (SEQ ID NO: 12) (10 μM, 1 μl)
· Amplification condition: 30 cycles of 98°C, 10 seconds; 55°C, 5 seconds; and 72°C, 1 minute

Two kinds of PCR products were each inserted, with use of NEBuilder HiFi DNA Assembly Master Mix (New England BioLabs), into a vector pTipQC1 (Qiagen) treated with restriction enzymes NcoI and HindIII. Next, E.coli JM109 was transformed with use of the plasmid pQAH-d803. The transformant was cultured at 28°C overnight in 2 ml of LB medium (including 50 µg/ml of ampicillin). pQAH-d803 was obtained from the bacterial cell, and then Rhodococcus erythropolis L88 (Hokkaido System Science) was transformed with use of the plasmid. The transformant was cultured at 28°C overnight in 2 ml of LB medium (including 20 µg/ml of chloramphenicol). Part of the culture solution was added to 2 ml of fresh LB medium (including 20 µg/ml of chloramphenicol), and was shake-cultured at 28°C. After the turbidity of the culture solution reached 0.8 (absorption wavelength: 600 nm), thiostrepton was added at a concentration of 0.2 µg/ml for the purpose of inducing expression of a protein. Next, 2-aminoisobutyric acid was added at a concentration of 10 mM as a reactive substrate. Shaking reaction was then started.

As a result, after 48 hours of reaction, production of α-methyl-D-serine was not determined in the culture supernatant.

The results of Example 3 and the above results show that hydroxylation activity expression requires the peg.803 protein.

### [Comparative Example 1] Evaluation of reactivity of transformed E. coli

A transformed E. coli that expresses the proteins with Protein ID peg.801-804 was developed as below.

Specifically, PCR was carried out under the PCR conditions below to obtain PCR products for peg.803, 804.

### <PCR conditions>

· Template: genome of R. wratislaviensis C31-06 strain (100 ng/µl, 1 µl)
· Polymerase: Tsk Gflex DNA Polymerase (Takara Bio Inc.) (1.25 units/µl, 1 µl)
· Primer (pQE60_5' Hyd): 
GAATTCATTAAAGAGGAGAAATTAACCATGGTTGCACCAACCTC
GAA (SEQ ID NO: 16) (10 μM, 1 μl)
· Primer (pQE60_3' Hyd): 
CAACAGGAGTCCAAGCTCAGCTAATTACTAGAGATCGAGGACG
AGCC (SEQ ID NO: 17) (10 μM, 1 μl)
· Amplification condition: 30 cycles of 98°C, 10 seconds; 58°C, 15 seconds; and 68°C, 1 minute

The PCR product was inserted, with use of NEBuilder HiFi DNA Assembly Master Mix (New England BioLabs), into a vector pQE60 (Qiagen) treated with restriction enzymes NcoI and HindIII. Next, E.coli JM109 was transformed with use of the plasmid pQEHyd. The transformant was cultured at 28°C overnight in 2 ml of LB medium (including 50 µg/ml of ampicillin). Next, part of the culture solution was added to 250 ml of TB medium (including 50 µg/ml of ampicillin), and was shake-cultured at 28°C. After the turbidity of the culture solution reached 0.6 (absorption wavelength: 600 nm), IPTG was added at a concentration of 1 mM for the purpose of inducing expression of a protein. After 16 hours of culture, 2-aminoisobutyric acid was added at a concentration of 10 mM as a reactive substrate. Shaking reaction was then started.

As a result, after 48 hours of reaction, production of 0.2 mM of α-methyl-D-serine was determined. This amount of production of α-methyl-D-serine was smaller than in Example 3.

This indicates that as a host for this reaction system, Rhodococcus bacteria allow higher reactivity than E. coli.

### [Example 5] Evaluation of reactivity resulting from introduction of peg.800

The protein obtained in Example 2 and having Protein ID peg.800 was expected, on the basis of the result of homology search, to be a transporter of 2-aminoisobutyric acid. Thus, a gene encoding the protein with Protein ID peg.800 was obtained as below.

Specifically, PCR was carried out under the PCR conditions below to obtain a PCR product for peg.800.

### <PCR conditions>

· Template: genome of R. wratislaviensis C31-06 strain (100 ng/µl, 1 µl)
· Polymerase: PrimeSTAR Max Premix (2x) (Takara Bio Inc.) (25 µl)
· Primer (pTipRT2_NdeI_5' 800): 
GTTTAACTTTAAGAAGGAGATATACATATGACAGTGAATCATTCC
CA (SEQ ID NO: 18) (10 μM, 1 μl)
· Primer (pTipRT2_HindIII_3' 800): 
TGGTGATGGTGATGCTCGAGAGATCTATCAGATTCTGGGCTGC
AGAA (SEQ ID NO: 19) (10 μM, 1 μl)
· Amplification condition: 30 cycles of 98°C, 10 seconds; 55°C, 5 seconds; and 72°C, 1 minute

The PCR product was inserted, with use of NEBuilder HiFi DNA Assembly Master Mix (New England BioLabs), into a vector pTipRT2 (Hokkaido System Science) treated with restriction enzymes NdeI and HindIII. Next, E.coli JM109 was transformed with use of the plasmid pRAT1. The transformant was cultured at 28°C overnight in 2 ml of LB medium (including 50 µg/ml of ampicillin). pRAT1 was obtained from the bacterial cell, and then pQAH1 Rhodococcus erythropolis L88 produced in Example 3 was transformed with use of the plasmid. The transformant (pQAH1/pRAT1 Rhodococcus erythropolis L88) was cultured at 28°C overnight in 2 ml of LB medium (including 5 µg/ml of tetracycline, 20 µg/ml of chloramphenicol). Part of the culture solution was added to 2 ml of fresh LB medium (including 5 µg/ml of tetracycline, 20 µg/ml of chloramphenicol), and was shake-cultured at 28°C. After the turbidity of the culture solution reached 0.8 (absorption wavelength: 600 nm), thiostrepton was added at a concentration of 0.2 µg/ml for the purpose of inducing expression of a protein. Next, 2-aminoisobutyric acid was added at a concentration of 10 mM as a reactive substrate. Shaking reaction was then started.

As a result, after 48 hours of reaction, production of 8.5 mM of α-methyl-D-serine was determined in the culture supernatant.

This Example resulted in determination of a larger amount of product than in Example 3. This indicates that the peg.800 protein improves the reactivity.

The α-methyl-D-serine produced had an optical purity of 93.5% e.e.

### <Analysis conditions>

· Measurement device: LCMS-2010A (Shimadzu Corporation)
· Column: Xbridge C18 column (5 µm: 2.1 × 150 mm) (Nihon Waters K.K.)
· Column oven temperature: 40°C
· Mobile phase A: 5% acetic acid
· Mobile phase B: acetonitrile/methanol = 90/10
· Flow rate: 0.25 ml/min
· Gradient setting: 0 to 0.1 min, 5% mobile phase B; 0.1 to 30 min, 5 to 35% mobile phase B; 30 to 40 min, 90% mobile phase B; 40 to 50 min, 5% mobile phase B
· MS condition: block temperature: 200°C, Curved desolvation line temperature: 250°C, Detector voltage: 1.5 kV, nebulizing gas flow: 1.51/min

The optical purity was analyzed with use of the product of mixing 25 µl of reaction solution with 25 µl of 0.8% triethylamine in acetonitrile solution and derivatizing the mixture with 50 µl of 2,3,4,6,-tetra-O-acetyl-β-D-glucopyranosyl isocyanate.

### [Example 6] Evaluation of substrate specificity

pQAH1/pRAT1 Rhodococcus erythropolis L88 was cultured in an LB medium by a method similar to that for Example 5. Expression of a protein was induced with 0.2 µg/ml of thiostrepton, and then 10 mM of each of L-isovaline, D-isovaline, L-aminobutyrate, and D-aminobutyrate was added together with 5% glucose. After 26 hours of reaction, the reaction supernatant was analyzed by the LCMS analysis method used for Example 1.

As a result, a product was determined of which the molecular weight was increased by 16 for each substrate. This indicates that a hydroxylation reaction was proceeding. The results of NMR and optical rotation analyses showed production of (2S,3S)-2-methyl threonine for a case where L-isovaline was a substrate, (2R,3R)-2-methyl threonine for a case where D-isovaline was a substrate, L-allo-threonine for a case where L-aminobutyrate was a substrate, and D-allo-threonine in a case where D-aminobutyrate was a substrate.

The results of NMR for (2S,3S)-2-methyl threonine and (2R,3R)-2-methyl threonine are as below (Table 3).

**[Table 3]**

| Product | Results of NMR |
|---|---|
| (2S,3S)-2-methyl threonine | [α]D²⁰=+8.8 (c 0.80, H2O); 1H NMR (D2O): δ 1.17 ( d, 3H, J = 6.6 Hz, CHOCH3), 1.46 [s, 3H, C(CH3) N], 4.02 (q, 1H, J = 6.6 Hz, CHOCH3); 13C NMR (D2O): δ 16.8, 20.0 [CHOCH3, C(CH3)N], 65.2 [C(C H3)N], 69.3 (CHOCH3), 175.1 (CO). |
| (2R,3R)-2-methyl threonine | 1H NMR (D2O): δ 1.18 (d, 3H, J = 6.6 Hz, CHOC H3), 1.47 [s, 3H, C (CH3)N], 4.03 (q, 1H, J = 6.6 Hz, CHOCH3); 13C NMR (D2O): δ 16.8, 20.0 [CH OCH3, C (CH3)N], 65.2 [C (CH3)N], 69.3 (CHOCH3) , 175.1 (CO). |

### [Example 7] Analysis of structure of peg.803, 804

A transformed E. coli that expresses the proteins with Protein ID peg.803, 804 was developed as below.

Specifically, PCR was carried out under the PCR conditions below to obtain PCR products for peg.803, 804.

### <PCR conditions>

- Template: genome of R. wratislaviensis C31-06 strain (100 ng/µl, 1 µl)
- Polymerase: PrimeSTAR Max Premix (2×) (Takara Bio Inc.) (25 µl)
- Primer (pQE_804_5'): 
TCGCATCACCATCACCATCACGGATCCATGGTTGCACCAACCTC
GAA (SEQ ID NO: 20) (10 μM, 1 μl)
- Primer (pQE_803_3'): 
CAACAGGAGTCCAAGCTCAGCTAATTATTAGTCCGCCTGATTCG
TAA (SEQ ID NO: 21) (10 μM, 1 μl)
- Amplification condition: 30 cycles of 98°C, 10 seconds; 55°C, 5 seconds; and 72°C, 1 minute

The PCR product was inserted, with use of NEBuilder HiFi DNA Assembly Master Mix (New England BioLabs), into a vector pQE80 (Qiagen) treated with restriction enzymes BamHI and HindIII. Next, E.coli Rosetta 2 (DE3) was transformed with use of the plasmid pQE804-803. The transformant was cultured at 28°C overnight in 2 ml of LB medium (including 50 µg/ml of ampicillin, 25 µg/ml of chloramphenicol). Part of the culture solution was added to 200 ml of TB medium (including 50 µg/ml of ampicillin, 25 µg/ml of chloramphenicol), and was shake-cultured at 20°C. After the turbidity of the culture solution reached 0.6 (absorption wavelength: 600 nm), IPTG was added at a concentration of 0.5 mM for the purpose of inducing expression of a protein. After 16 hours of culture, the bacterial cell was recovered through centrifugation, and was washed twice with 0.85% NaCl. The bacterial cell was suspended in 0.5 M of NaCl, 30 mM of imidazole, and 20 mM of HEPES (pH 8.0), and was homogenized ultrasonically. The supernatant of the homogenate as centrifuged was introduced into HisTALON Superflow (1.6 × 2.5 cm) (Takara Bio Inc.), and was eluted with use of 0.5 M of NaCl, 30 mM of imidazole, and 150 mM of HEPES (pH 8.0) for recovery of an enzyme fraction including peg.803, 804. The enzyme fraction was concentrated through ultrafiltration, and was then introduced into a Superdex 200 Increase 10/300GL column (1.0 × 30 cm) (GE Healthcare). Then, the respective molecular weights of the proteins with peg.803, 804 were measured at approximately 180,000 with use of 10 mM of HEPES (pH 8.0) and 0.14M of NaCl. The respective assumed molecular weights of the proteins with peg.803, 804 are 42,000 and 43,000, respectively. This result and the results of the SDS-PAGE analysis indicate that the proteins with peg.803, 804 are associated with each other in an equimolar manner.

This suggests that the monooxygenase includes the proteins with peg.803 and peg.804 and has a heterotetramer structure as a whole.

### [Example 8] Identification of coenzyme of peg.801

A transformed E. coli that expresses the protein with Protein ID peg.801 was developed as below.

Specifically, PCR was carried out under the PCR conditions below to obtain a PCR product for peg.801.

### <PCR conditions>

- Template: genome of R. wratislaviensis C31-06 strain (100 ng/µl, 1 µl)
- Polymerase: PrimeSTAR Max Premix (2×) (Takara Bio Inc.) (25 µl)
- Primer (pQE_801_5'): 
TCGCATCACCATCACCATCACGGATCCATGACCAATTCAGATAG

TTC (SEQ ID NO: 22) (10 μM, 1 μl)
- Primer (pQE_801_3'): 
CAACAGGAGTCCAAGCTCAGCTAATTACTAGAGATCGAGGACG
AGCC (SEQ ID NO: 23) (10 μM, 1 μl)
- Amplification condition: 30 cycles of 98°C, 10 seconds; 55°C, 5 seconds; and 72°C, 1 minute

The PCR product was inserted, with use of NEBuilder HiFi DNA Assembly Master Mix (New England BioLabs), into a vector pQE80 (Qiagen) treated with restriction enzymes BamHI and HindIII. Next, E.coli Rosetta 2 (DE3) was transformed with use of the plasmid pQE-801. The transformant was cultured at 28°C overnight in 2 ml of LB medium (including 50 µg/ml of ampicillin, 25 µg/ml of chloramphenicol). Part of the culture solution was added to 200 ml of TB medium (including 50 µg/ml of ampicillin, 25 µg/ml of chloramphenicol), and was shake-cultured at 20°C. After the turbidity of the culture solution reached 0.6 (absorption wavelength: 600 nm), IPTG was added at a concentration of 0.5 mM for the purpose of inducing expression of a protein. After 16 hours of culture, the bacterial cell was recovered through centrifugation, and was washed twice with 0.85% NaCl. The bacterial cell was suspended in 0.5 M of NaCl, 30 mM of imidazole, and 20 mM of HEPES (pH 8.0), and was crushed ultrasonically. The supernatant of the crushed liquid as centrifuged was introduced into HisTALON Superflow (1.6 × 2.5 cm) (Takara Bio Inc.), and was eluted with use of 0.5 M of NaCl, 30 mM of imidazole, and 150 mM of HEPES (pH 8.0) for recovery of an enzyme fraction including peg.801. The enzyme fraction was concentrated through ultrafiltration, was then introduced into a MonoQ 10/ 100 GL column (1.0 × 10 cm) (GE healthcare), and was eluted with use of 1M of NaCl and 20 mM of Tris-HCl buffer (pH 7.4). This produced a purified enzyme of peg.801. Then, 0.77 µg/ml of the purified enzyme of peg.801 was added to a reaction solution including 10-150 µM of NADH or NADPH, 20-100 µM of dichloroindophenol, and 100 mM of a potassium phosphate buffer (pH 7.5) for measurement of the enzyme activity of the purified enzyme of peg.801.

The Km value for this reaction was measured at 8.2 µM for NADH and 6.2 mM for NADPH. This shows that peg.801 requires NADH as a coenzyme.

### Industrial Applicability

The present invention is applicable to fields that require stable protein supplies, for example, the field of peptide medicine production.

### [Accession number]

NITE BP-02370

## Claims

1. A monooxygenase, comprising:
two kinds of hetero subunits.

2. The monooxygenase according to claim 1, wherein
the monooxygenase catalyzes a reaction for converting an α-amino acid or α,α-disubstituted amino acid into a β-hydroxyamino acid.

3. The monooxygenase according to claim 2, wherein
the α-amino acid or α,α-disubstituted amino acid is a compound represented by Formula (1) below: 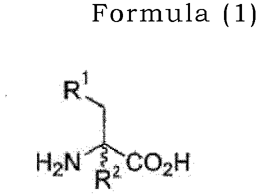 where R¹ and R² are each independently a hydrogen or CH₃, and
the β-hydroxyamino acid is a compound represented by Formula (2) below: 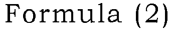 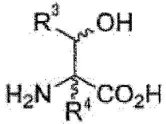 where R³ and R⁴ are each independently a hydrogen or CH₃.

4. The monooxygenase according to any one of claims 1 to 3, wherein
the monooxygenase catalyzes at least one of respective reactions represented by Formulae (3) to (7) below which reactions are each for converting the α-amino acid or α,α-disubstituted amino acid into the β-hydroxyamino acid,

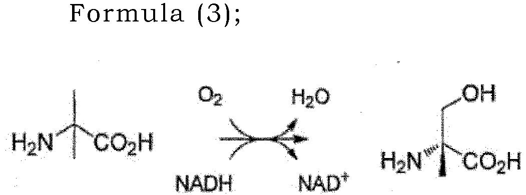

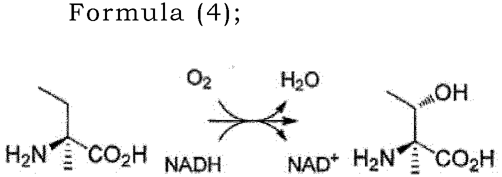

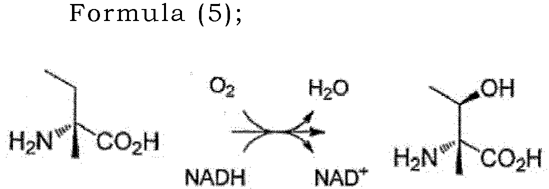

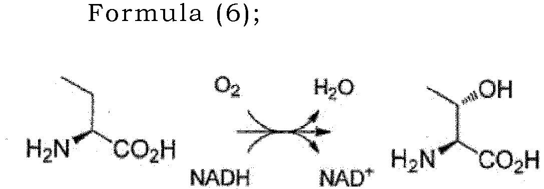

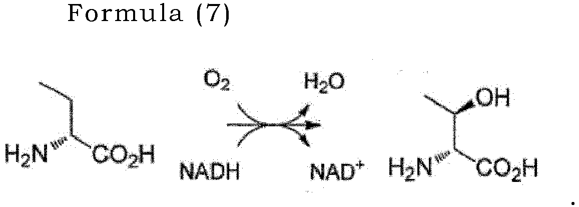

5. The monooxygenase according to any one of claims 1 to 4, wherein
assuming that the two kinds of hetero subunits are an α subunit and a β subunit,
the α subunit includes a protein selected from the group consisting of (a) to (d) below, and
the β subunit includes a protein selected from the group consisting of (e) to (h) below,
(a) a protein consisting of an amino acid sequence represented by SEQ ID NO: 1;
(b) a protein derived from the amino acid sequence of SEQ ID NO: 1 by substitution, deletion, insertion, and/or addition of one (1) or several amino acid residues, a subunit including the protein (b) having monooxygenase activity in a case where the subunit including the protein (b) has formed a complex together with the β subunit;
(c) a protein consisting of an amino acid sequence having a homology of not less than 80% with respect to the amino acid sequence represented by SEQ ID NO: 1, a subunit including the protein (c) having monooxygenase activity in a case where the subunit including the protein (c) has formed a complex together with the β subunit;
(d) a protein encoded by a gene consisting of a base sequence represented by SEQ ID NO: 6;
(e) a protein consisting of an amino acid sequence represented by SEQ ID NO: 2;
(f) a protein derived from the amino acid sequence of SEQ ID NO: 2 by substitution, deletion, insertion, and/or addition of one (1) or several amino acid residues, a subunit including the protein (f) having monooxygenase activity in a case where the subunit including the protein (f) has formed a complex together with the α subunit;
(g) a protein consisting of an amino acid sequence having a homology of not less than 80% with respect to the amino acid sequence represented by SEQ ID NO: 2, a subunit including the protein (g) having monooxygenase activity in a case where the subunit including the protein (g) has formed a complex together with the α subunit, and
(h) a protein encoded by a gene consisting of a base sequence represented by SEQ ID NO: 7.

6. A monooxygenase reaction system for producing a β-hydroxyamino acid,
the system comprising:
a monooxygenase according to any one of claims 1 to 5; and an electron transport chain protein.

7. The monooxygenase reaction system according to claim 6, wherein
the electron transport chain protein includes a protein selected from the group consisting of (i) to (1) below and a protein selected from the group consisting of (m) to (p) below,
(i) a protein consisting of an amino acid sequence represented by SEQ ID NO: 3;
(j) a protein derived from the amino acid sequence of SEQ ID NO: 3 by substitution, deletion, insertion, and/or addition of one (1) or several amino acid residues, the protein (j) having electron transport activity in a case where the protein (j) has been combined with a protein selected from the group consisting of (m) to (p) below;
(k) a protein consisting of an amino acid sequence having a homology of not less than 80% with respect to the amino acid sequence represented by SEQ ID NO: 3, the protein (k) having electron transport activity in a case where the protein (k) has been combined with a protein selected from the group consisting of (m) to (p) below;
(1) a protein encoded by a gene consisting of a base sequence represented by SEQ ID NO: 8;
(m) a protein consisting of an amino acid sequence represented by SEQ ID NO: 4;
(n) a protein derived from the amino acid sequence of SEQ ID NO: 4 by substitution, deletion, insertion, and/or addition of one (1) or several amino acid residues, the protein (n) having electron transport activity in a case where the protein (n) has been combined with a protein selected from the group consisting of (i) to (1) above;
(o) a protein consisting of an amino acid sequence having a homology of not less than 80% with respect to the amino acid sequence represented by SEQ ID NO: 4, the protein (o) having electron transport activity in a case where the protein (o) has been combined with a protein selected from the group consisting of (i) to (1) above; and
(p) a protein encoded by a gene consisting of a base sequence represented by SEQ ID NO: 9.

8. A monooxygenase gene encoding a protein recited in any one of claims 1 to 5.

9. A recombinant vector, comprising:
a gene according to claim 8.

10. A transformant, comprising:
a gene according to claim 8; or a recombinant vector according to claim 9.

11. The transformant according to claim 10, further comprising:
(1) a gene encoding an electron transport chain protein; and/or
(2) a gene encoding a transporter protein.

12. The transformant according to claim 11, wherein
the electron transport chain protein includes a protein selected from the group consisting of (i) to (1) below and a protein selected from the group consisting of (m) to (p) below,
(i) a protein consisting of an amino acid sequence represented by SEQ ID NO: 3;
(j) a protein derived from the amino acid sequence of SEQ ID NO: 3 by substitution, deletion, insertion, and/or addition of one (1) or several amino acid residues, the protein (j) having electron transport activity in a case where the protein (j) has been combined with a protein selected from the group consisting of (m) to (p) below;
(k) a protein consisting of an amino acid sequence having a homology of not less than 80% with respect to the amino acid sequence represented by SEQ ID NO: 3, the protein (k) having electron transport activity in a case where the protein (k) has been combined with a protein selected from the group consisting of (m) to (p) below;
(1) a protein encoded by a gene consisting of a base sequence represented by SEQ ID NO: 8;
(m) a protein consisting of an amino acid sequence represented by SEQ ID NO: 4;
(n) a protein derived from the amino acid sequence of SEQ ID NO: 4 by substitution, deletion, insertion, and/or addition of one (1) or several amino acid residues, the protein (n) having electron transport activity in a case where the protein (n) has been combined with a protein selected from the group consisting of (i) to (1) above;
(o) a protein consisting of an amino acid sequence having a homology of not less than 80% with respect to the amino acid sequence represented by SEQ ID NO: 4, the protein (o) having electron transport activity in a case where the protein (o) has been combined with a protein selected from the group consisting of (i) to (1) above; and
(p) a protein encoded by a gene consisting of a base sequence represented by SEQ ID NO: 9.

13. The transformant according to claim 11 or 12, wherein
the transporter protein is a protein selected from the group consisting of (q) to (t) below,
(q) a protein consisting of an amino acid sequence represented by SEQ ID NO: 5;
(r) a protein derived from the amino acid sequence of SEQ ID NO: 5 by substitution, deletion, insertion, and/or addition of one (1) or several amino acid residues, the protein (r) having transport activity;
(s) a protein consisting of an amino acid sequence having a homology of not less than 80% with respect to the amino acid sequence represented by SEQ ID NO: 5, the protein (s) having transport activity; and
(t) a protein encoded by a gene consisting of a base sequence represented by SEQ ID NO: 10.

14. The transformant according to any one of claims 10 to 13, wherein
the transformant is a Rhodococcus bacterium.

15. A method for producing a β-hydroxyamino acid, the method comprising the step of:
culturing a transformant according to any one of claims 10 to 14 in a medium including an α-amino acid or an α,α-disubstituted amino acid.

16. The method according to claim 15, wherein
a gene according to claim 8 which gene is included in the transformant is derived from Rhodococcus.

17. A Rhodococcus wratislaviensis C31-06 strain with an accession number of NITE BP-02370.
